(19) Europäisches Patentamt
European Patent Office
Office européen des brevets

(11) EP 3 439 057 A1

(12) **EUROPEAN PATENT APPLICATION**
published in accordance with Art. 153(4) EPC

(43) Date of publication:
**06.02.2019 Bulletin 2019/06**

(21) Application number: **17773763.2**

(22) Date of filing: **15.02.2017**

(51) Int Cl.:
***H01L 51/46*** (2006.01) ***H01L 51/44*** (2006.01)
***C07D 495/04*** (2006.01)

(86) International application number:
**PCT/JP2017/005393**

(87) International publication number:
**WO 2017/169215 (05.10.2017 Gazette 2017/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **31.03.2016 JP 2016072734**

(71) Applicant: **Fujifilm Corporation**
**Minato-ku**
**Tokyo 106-8620 (JP)**

(72) Inventors:
- **SATO, Hirotaka**
**Ashigarakami-gun**
**Kanagawa 258-8577 (JP)**
- **SHIROKANE, Kenji**
**Ashigarakami-gun**
**Kanagawa 258-8577 (JP)**

(74) Representative: **Hoffmann Eitle**
**Patent- und Rechtsanwälte PartmbB**
**Arabellastraße 30**
**81925 München (DE)**

(54) **PHOTOELECTRIC CONVERSION ELEMENT, SOLAR CELL, AND COMPOUND**

(57) Provided is a photoelectric conversion element in which a moisture resistance variation is suppressed, a solar cell including the photoelectric conversion element, and a new compound that is appropriately used in the photoelectric conversion element. A photoelectric conversion element includes, at least: a conductive support; a photosensitive layer that contains a light absorbing agent; a hole transport layer that contains a hole transporting material; and a second electrode, in which at least one of the photosensitive layer or the hole transport layer is provided on the conductive support to constitute a first electrode in combination with the conductive support, and in which the hole transport layer contains a compound Q having at least one structure M represented by Formula 1 as the hole transporting material, provided that in a case where the compound Q has two or more of the structures M, the two or more structures M may be the same as or different from each other.

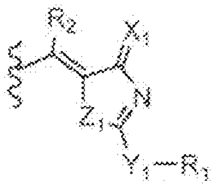

Formula 1

EP 3 439 057 A1

FIG. 1A

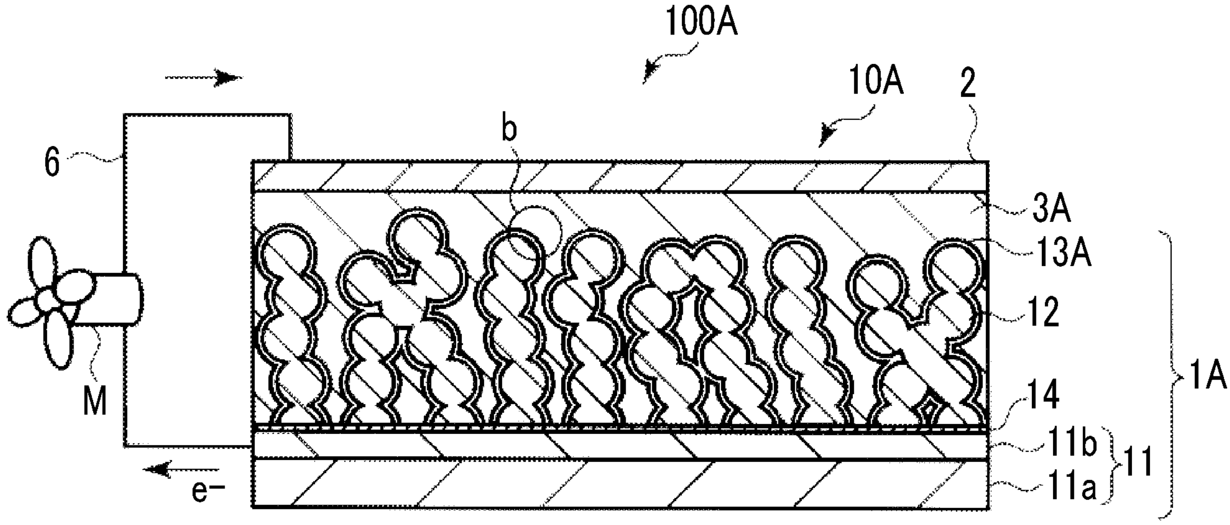

## Description

## BACKGROUND OF THE PRESENT INVENTION

1. Field of the present invention

The present invention relates to a photoelectric conversion element, a solar cell, and a compound.

2. Description of the Related Art

Photoelectric conversion elements are used in a variety of optical sensors, copiers, solar cells, and the like. It is expected that solar cells will be actively put into practical use as cells using non-exhaustible solar energy. Examples of the type of the solar cells include a silicon type, a dye sensitized type, an organic thin film, and the like (for example, JP2013-109965A). Among these, research and development of dye sensitized solar cells, in which an organic dye, a Ru bipyridyl complex, or the like is used as a sensitizer, are actively in progress, and the photoelectric conversion efficiency thereof reaches approximately 11%.

Meanwhile, in recent years, there have been reported research results indicating that solar cells using a metal halide as a compound (hereinafter, also referred to as "perovskite compound" or "perovskite-type light absorbing agent") having a perovskite-type crystal structure are capable of achieving relatively high photoelectric conversion efficiency, and the solar cells attract attention.

For example, a solar cell using a compound having a perovskite-type crystal structure of $CH_3NH_3PbI_2Cl$, and a hole transporting material is described in Science 388, 2012, p. 643 to 647.

In addition, a solar cell using an A-D-A-type compound that is a composite of an acceptor (A) and a donor (D) as a hole transporting material is described in J. Mater. Chem. A, 2015, 3, p. 11940 to 11947, and Energy Environ. Sci., 2014, 7, p. 2981 to 2985.

## SUMMARY OF THE PRESENT INVENTION

In a photoelectric conversion element or a solar cell which uses a perovskite compound, constant achievement is attained in an improvement of photoelectric conversion efficiency. However, the photoelectric conversion element or the solar cell which uses the perovskite compound has attracted attention in recent years, and little is known about battery performance other than photoelectric conversion efficiency.

The photoelectric conversion element and the solar cell are required to have durability capable of maintaining initial performance in a field circumstance in which the photoelectric conversion element and the solar cell are actually used in addition to high photoelectric conversion efficiency.

However, it is known that the perovskite compound is easily damaged under a high-humidity environment or a high-humidity and heat environment. Actually, in a photoelectric conversion element or a solar cell which uses the perovskite compound as a light absorbing agent, photoelectric conversion efficiency greatly deteriorates under the high-humidity environment or the high-humidity and heat environment in many cases. In addition, a problem of a moisture resistance variation is also pointed out.

With regard to a cause for generation of the variation in the moisture resistance, it is assumed that the perovskite compound is decomposed due to intrusion of water from a defect in an interface between the perovskite compound and the hole transporting material or moisture that is contained in the hole transporting material. In J. Mater. Chem. A, 2015, 3, p. 11940 to 11947, and Energy Environ. Sci., 2014, 7, p. 2981 to 2985, an A-D-A type hole transporting material that does not uses a hydrophilic additive (LiTFSI and the like) is used. From a result obtained through extensive study made by the present inventors, it could be understood that the moisture resistance variation can be suppressed to a certain extent in a case of using the hole transporting material, but the suppression is not sufficient. The reason for this is considered to be because it is difficult to suppress intrusion of water from a defect in a material interface and the like.

The present invention has been made in consideration of the above-described circumstances, and an object thereof is to provide a photoelectric conversion element in which a moisture resistance variation is suppressed, particularly, a photoelectric conversion element which uses a perovskite compound as a light absorbing agent in a photosensitive layer and in which a moisture resistance variation is suppressed. In addition, another object of the present invention is to provide a solar cell including the photoelectric conversion element. In addition, still another object of the present invention is to provide a new compound that is appropriately used in the photoelectric conversion element.

The present inventors and the like have developed the new compound having at least one specific partial structure, and have found that, when using the new compound in the photoelectric conversion element or the solar cell as the hole transporting material, the moisture resistance variation is improved. Furthermore, the present inventors and the like have found that the effect of suppressing the moisture resistance variation by using this new compound as a

hole transporting material is exhibited particularly in the photoelectric conversion element and the solar cell which use the perovskite compound as the light absorbing agent.

That is, the objects of the present invention have been solved by the following means, which are aspects of the present invention.

[1] A photoelectric conversion element comprising, at least: a conductive support; a photosensitive layer that contains a light absorbing agent; a hole transport layer that contains a hole transporting material; and a second electrode, in which at least one of the photosensitive layer or the hole transport layer is provided on the conductive support to constitute a first electrode in combination with the conductive support, and in which the hole transport layer contains at least a compound Q having at least one structure M represented by Formula 1 as the hole transporting material, provided that in a case where the compound Q has two or more of the structures M, the two or more structures M may be the same as or different from each other.

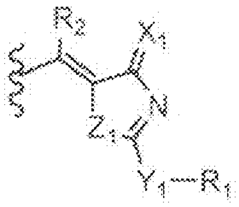

Formula 1

In Formula 1, $Z_1$ represents a sulfur atom, a selenium atom, or an oxygen atom, $Y_1$ represents NR, a sulfur atom, a selenium atom, or an oxygen atom, $X_1$ represents NR, a sulfur atom, an oxygen atom, a selenium atom, or $CR_3R_4$, $R_1$ represents a substituent, $R_2$, $R_3$, $R_4$ and R each independently represent a hydrogen atom or a substituent, and a wavy line represents a binding site with a remainder of the compound Q.

[2] The photoelectric conversion element according to [1], in which the hole transport layer contains at least a compound Q represented by Formula 2 as the hole transporting material.

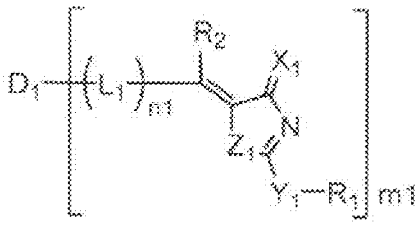

Formula 2

In Formula 2, $Z_1$ represents a sulfur atom, a selenium atom, or an oxygen atom, $Y_1$ represents NR, a sulfur atom, a selenium atom, or an oxygen atom, $X_1$ represents NR, a sulfur atom, an oxygen atom, a selenium atom, or $CR_3R_4$, $R_1$ represents a substituent, and $R_2$, $R_3$, $R_4$ and R each independently represent a hydrogen atom or a substituent.

$L_1$ represents a linking group.

$D_1$ represents an organic group or a nitrogen atom. The organic group represented by $D_1$ may have another substituent in Formula 2 that is different from a substituent represented by Formula 2a, and the nitrogen atom may have another substituent that is different from a substituent represented by Formula 2a or a hydrogen atom. $Z_1$, $Y_1$, $X_1$, $R_1$, $R_2$, $L_1$, n1, and m1 in Formula 2a are the same as $Z_1$, $Y_1$, $X_1$, $R_1$, $R_2$, $L_1$, n1, and m1 in Formula 2.

n1 represents an integer of 0 to 11. In a case where n1 is an integer of 2 or more, a plurality of $L_1$'s may be the same as or different from each other.

In a case where $D_1$ is an organic group, m1 represents an integer of 1 to 4, and in a case where $D_1$ is a nitrogen atom, m1 represents an integer of 1 to 3. In a case where m1 is an integer of 2 or more, a plurality of $Z_1$'s, $Y_1$'s, $X_1$'s, $R_1$'s, and $R_2$'s may be the same as or different from each other, respectively.

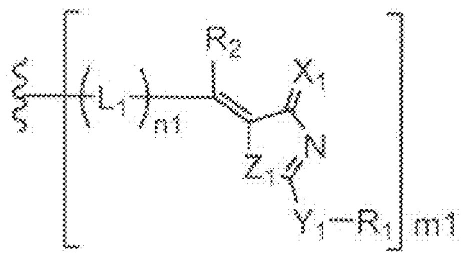

Formula 2a

[3] The photoelectric conversion element according to [1] or [2], in which the hole transport layer contains at least a compound Q represented by Formula 3 as the hole transporting material.

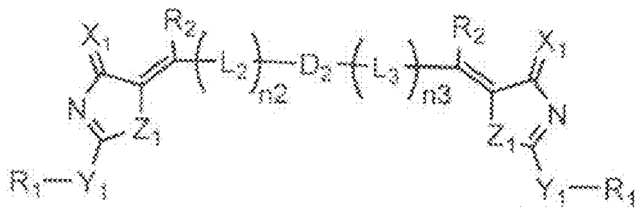

Formula 3

In Formula 3, $Z_1$'s each independently represent a sulfur atom, a selenium atom, or an oxygen atom, $Y_1$'s each independently represent NR, a sulfur atom, a selenium atom, or an oxygen atom, $X_1$'s each independently represent NR, a sulfur atom, an oxygen atom, a selenium atom, or $CR_3R_4$, $R_1$'s each independently represent a substituent, $R_2$, $R_3$, $R_4$, and R each independently represent a hydrogen atom or a substituent, $L_2$ and $L_3$ each independently represent an alkenylene group, a cycloalkenylene group, an alkynylene group, an arylene group, or a heteroarylene group, $D_2$ represents a linking group, n2 and n3 each independently represent an integer of 1 to 5, and in a case where n2 is an integer of 2 to 5, a plurality of $L_2$'s may be the same as or different from each other, and in a case where n3 is an integer of 2 to 5, a plurality of $L_3$'s may be the same as or different from each other.

[4] The photoelectric conversion element according to [3], in which $D_2$ in Formula 3 represents a divalent fused ring group containing at least one aromatic ring or a divalent fused ring group containing at least one aromatic hetero ring.

[5] The photoelectric conversion element according to any one of [1] to [4], in which the at least one $X_1$ is an oxygen atom.

[6] The photoelectric conversion element according to any one of [1] to [5], in which the at least one $Z_1$ is a sulfur atom.

[7] The photoelectric conversion element according to any one of [1] to [6], in which the at least one $Y_1$ is a sulfur atom.

[8] The photoelectric conversion element according to any one of [1] to [7], in which the photosensitive layer contains at least a compound having a perovskite-type crystal structure that has a cation of an element of Group 1 in the periodic table or a cationic organic group A, a cation of a metal atom M other than elements of Group 1 in the periodic table, and an anion of an anionic atom or atomic group X as the light absorbing agent.

[9] A solar cell, comprising the photoelectric conversion element according to any one of [1] to [8].

[10] A compound represented by Formula 3.

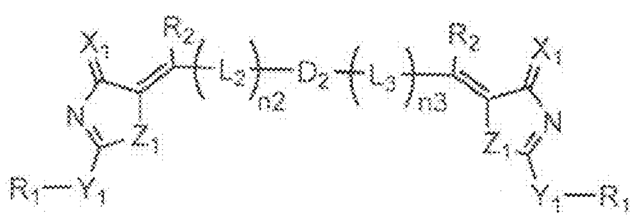

Formula 3

In Formula 3, $Z_1$'s each independently represent a sulfur atom, a selenium atom, or an oxygen atom, $Y_1$'s each independently represent NR, a sulfur atom, a selenium atom, or an oxygen atom, $X_1$'s each independently represent NR, a sulfur atom, an oxygen atom, a selenium atom, or $CR_3R_4$, $R_1$'s each independently represent a substituent, $R_2$, $R_3$, $R_4$, and R each independently represent a hydrogen atom or a substituent, $L_2$ and $L_3$ each independently represent an alkenylene group, a cycloalkenylene group, an alkynylene group, an arylene group, or a heteroarylene group, $D_2$ represents a linking group, n2 and n3 each independently represent an integer of 1 to 5, and in a case where n2 is an integer of 2 to 5, a plurality of $L_2$'s may be the same as or different from each other, and in a case where n3 is an integer of 2 to 5, a plurality of $L_3$'s may be the same as or different from each other.

[11] The compound according to [10], in which $D_2$ in Formula 3 represents a divalent fused ring group containing at least one aromatic ring or a divalent fused ring group containing at least one aromatic hetero ring.

In this specification, parts of respective formulae may be expressed as a rational formula for understanding of chemical structures of compounds. According to this, in the respective formulae, partial structures are called (substituent) groups, ions, atoms, and the like, but in this specification, the partial structures may represent an atomic group or elements which constitute (substituent) groups or ions represented by the formulae in addition to the (substituent) groups, the ions, the atoms, and the like.

In this specification, with regard to expression of compounds (including a complex and a dye), the expression is also used to indicate salts of the compounds and ions of the compounds in addition to the compounds. In addition, the compounds include compounds of which a partial structure is changed in a range exhibiting a target effect. In addition, with regard to compounds for which substitution or non-substitution is not specified, the compounds also include compounds which have an arbitrary substituent in a range exhibiting a desired effect. This is also true of substituents, linking groups, and the like (hereinafter, referred to as "substituents and the like").

In this specification, in a case where a plurality of substituents and the like expressed using specific symbols or a plurality of substituents and the like are simultaneously defined, the respective substituents and the like may be identical to or different from each other unless otherwise stated. This is also true of definition of the number of substituents and the like. In addition, in a case of approaching each other (particularly, in a case of being close to each other), the plurality of substituents and the like may be bonded to each other to form a ring unless otherwise stated. In addition, rings, for example, alicycles, aromatic rings, and hetero rings may be additionally fused together to form a fused ring.

In this specification, numerical ranges represented by using "to" include ranges including numerical values before and after "to" as the lower limit and the upper limit.

According to the aspects of the present invention, it is possible to provide a photoelectric conversion element in which a moisture resistance variation is suppressed, and a solar cell including the photoelectric conversion element. In addition, according to the present invention, it is possible to provide a new compound that is used as the hole transporting material in the photoelectric conversion element, thereby leading to an improvement in the moisture resistance variation.

## BRIEF DESCRIPTION OF THE DRAWINGS

Fig. 1A is a cross-sectional view schematically illustrating a preferred aspect of a photoelectric conversion element of the present invention.

Fig. 1B is an enlarged view of a cross-sectional region b in Fig. 1A.

Fig. 2 is a cross-sectional view schematically illustrating another preferred aspect of the photoelectric conversion element of the present invention.

Fig. 3 is a cross-sectional view schematically illustrating still another preferred aspect of the photoelectric conversion element of the present invention.

Fig. 4 is a cross-sectional view schematically illustrating still another preferred aspect of the photoelectric conversion element of the present invention.

Fig. 5 is a cross-sectional view schematically illustrating still another preferred aspect of the photoelectric conversion element of the present invention.

## DESCRIPTION OF THE PREFERRED EMBODIMENTS

<<Photoelectiic Conversion Element>>

A photoelectric conversion element of the present invention includes at least a conductive support, a photosensitive layer that contains a light absorbing agent, a hole transport layer that contains a hole transporting material, and a second electrode. Here, at least one of the photosensitive layer or the hole transport layer is provided on the conductive support to constitute the first electrode in combination with the conductive support.

In the present invention, the aspect in which the photosensitive layer or the hole transport layer is provided on the conductive support includes an aspect in which the photosensitive layer or the hole transport layer is provided (directly provided) to be in contact with a surface of the conductive support, and an aspect in which the photosensitive layer or the hole transport layer is provided on an upper side of the surface of the conductive support through another layer.

In the aspect in which the photosensitive layer or the hole transport layer is provided on the upper side of the surface of the conductive support through another layer, as the other layer that is provided between the conductive

support and the photosensitive layer or the hole transport layer, there is no particular limitation as long as the other layer does not deteriorate a battery performance of a solar cell. Examples of the other layer include a porous layer, a blocking layer, an electron transport layer, and the like.

In the present invention, examples of the aspect in which the photosensitive layer is provided on the upper side of the surface of the conductive support through another layer includes an aspect in which the photosensitive layer is provided on a surface of the porous layer in a thin film shape (refer to Fig. 1A and Fig. 1B), an aspect in which the photosensitive layer is provided on the surface of the porous layer in a thick film shape (refer to Fig. 2), an aspect in which the photosensitive layer is provided on a surface of the blocking layer in a thin film shape or a thick film shape (refer to Fig. 3), an aspect in which the photosensitive layer is provided on a surface of the electron transport layer in a thin film shape or a thick film shape (refer to Fig. 4), an aspect in which the photosensitive layer is provided on a surface of the hole transport layer in a thin film shape or a thick film shape (refer to Fig. 5), and the like. The photosensitive layer may be provided in a linear shape or in a dispersed pattern, but is preferably provided in a film shape.

In the photoelectric conversion element of the present invention, a configuration other than a configuration defined in the present invention is not particularly limited, and it is possible to employ a configuration that is known with respect to the photoelectric conversion element and a solar cell. Respective layers, which constitute the photoelectric conversion element of the present invention, are designed in correspondence with the purposes thereof, and may be formed, for example, in a monolayer or multilayers. For example, the porous layer may be provided between the conductive support and the photosensitive layer (refer to Fig. 1A and Fig. 2).

The photoelectric conversion element of the present invention contains a new compound (Q) having at least one specific partial structure to be described later as the hole transporting material to be contained in the hole transport layer. In a case where the photoelectric conversion element is used in various solar cells, for example, perovskite-type solar cells, dye sensitized solar cells, organic thin film solar cells, and the like, the moisture resistance variation can be suppressed.

Hereinafter, as a preferable aspect of the photoelectric conversion element of the present invention, the photoelectric conversion element having the photosensitive layer that contains the perovskite-type light absorbing agent will be described.

In Fig. 1A, Fig. 1B, and Fig. 2 to Fig. 5, the same reference numeral represents the same constituent element (member).

Furthermore, in Fig. 1A and Fig. 2, the size of fine particles which form a porous layer 12 is illustrated in a highlighted manner. These fine particles are preferably packed with each other (are vapor-deposited or in close contact with each other) in the horizontal direction and the vertical direction with respect to a conductive support 11 to form a porous structure.

In this specification, simple description of "photoelectric conversion element 10" represents photoelectric conversion elements 10A to 10E unless otherwise stated. This is also true of a system (hereinafter, referred to as "system") 100 that uses a solar cell and a first electrode 1. In addition, simple description of "photosensitive layer 13" represents photosensitive layers 13A to 13C unless otherwise stated. Similarly, description of "hole transport layer 3" represents hole transport layers 3A and 3B unless otherwise stated.

Examples of a preferred aspect of the photoelectric conversion element of the present invention include the photoelectric conversion element 10A illustrated in Fig. 1A. A system 100A illustrated in Fig. 1A is a system in which the photoelectric conversion element 10A is applied to a cell that allows operation means M (for example, an electric motor) to operate with an external circuit 6.

The photoelectric conversion element 10A includes a first electrode 1A, a second electrode 2, and a hole transport layer 3A.

The first electrode 1A includes a conductive support 11 including a support 11a and a transparent electrode 11b, a porous layer 12, and a photosensitive layer 13A that is provided as a perovskite-type light absorbing agent on a surface of the porous layer 12 as schematically illustrated in Fig. 1B in which a cross-sectional region b in Fig. 1A is enlarged. A blocking layer 14 is provided on the transparent electrode 11b, and the porous layer 12 is formed on the blocking layer 14. As described above, in the photoelectric conversion element 10A including the porous layer 12, it is assumed that a surface area of the photosensitive layer 13A increases, and thus charge separation and charge migration efficiency are improved.

The photoelectric conversion element 10B illustrated in Fig. 2 schematically illustrates a preferred aspect in which the photosensitive layer 13A of the photoelectric conversion element 10A illustrated in Fig. 1A is provided to be thick. In the photoelectric conversion element 10B, a hole transport layer 3B is provided to be thin. The photoelectric conversion element 10B is different from the photoelectric conversion element 10A illustrated in Fig. 1A in the film thickness of the photosensitive layer 13B and the hole transport layer 3B, but the photoelectric conversion element 10B has the same configuration as that of the photoelectric conversion element 10A except for the difference.

The photoelectric conversion element 10C illustrated in Fig. 3 schematically illustrates another preferred aspect of the photoelectric conversion element of the present invention. The photoelectric conversion element 10C is different

from the photoelectric conversion element 10B illustrated in Fig. 2 in that the porous layer 12 is not provided, but the photoelectric conversion element 10C has the same configuration as that of the photoelectric conversion element 10B except for the difference. That is, in the photoelectric conversion element 10C, the photosensitive layer 13C is formed on the surface of the blocking layer 14 in a thick film shape. In the photoelectric conversion element 10C, the hole transport layer 3B may be provided to be thick in the same manner as in the hole transport layer 3A illustrated in Fig. 1A.

The photoelectric conversion element 10D illustrated in Fig. 4 schematically illustrates still another preferred aspect of the photoelectric conversion element of the present invention. The photoelectric conversion element 10D is different from the photoelectric conversion element 10C illustrated in Fig. 3 in that an electron transport layer 15 is provided instead of the blocking layer 14, but the photoelectric conversion element 10D has the same configuration as that of the photoelectric conversion element 10C except for the difference. The first electrode 1D includes the conductive support 11, and the electron transport layer 15 and the photosensitive layer 13C which are sequentially formed on the conductive support 11. The photoelectric conversion element 10D is preferable when considering that the respective layers can be formed from an organic material. According to this, the productivity of the photoelectric conversion element is improved, and thickness reduction or flexibilization becomes possible.

The photoelectric conversion element 10E illustrated in Fig. 5 schematically illustrates still another preferred aspect of the photoelectric conversion element of the present invention. A system 100E including the photoelectric conversion element 10E is a system that is applied to battery use in the same manner as in the system 100A.

The photoelectric conversion element 10E includes a first electrode 1E, a second electrode 2, and an electron transport layer 4 that is provided between the first electrode 1E and the second electrode 2. The first electrode 1E includes the conductive support 11, and a hole transport layer 16 and the photosensitive layer 13C which are formed on the conductive support 11 in this order. The photoelectric conversion element 10E is preferable when considering that respective layers can be formed from an organic material in the same manner as in the photoelectric conversion element 10D.

In the present invention, a system 100 to which the photoelectric conversion element 10 is applied functions as a solar cell as follows.

Specifically, in the photoelectric conversion element 10, light that is transmitted through the conductive support 11, or the second electrode 2 and is incident to the photosensitive layer 13 excites a light absorbing agent. The excited light absorbing agent includes high-energy electrons and can emit the electrons. The light absorbing agent, which emits high-energy electrons, becomes an oxidized substance.

In the photoelectric conversion elements 10A to 10D, electrons emitted from the light absorbing agent migrate between a plurality of the light absorbing agents and reach the conductive support 11. The electrons which have reached the conductive support 11 work in the external circuit 6, and then return to the photosensitive layer 13 through the second electrode 2 and the hole transport layer 3. The light absorbing agent is reduced by the electrons which have returned to the photosensitive layer 13.

As described above, in the photoelectric conversion element 10, a cycle of excitation of the light absorbing agent and electron migration is repeated, and thus the system 100 functions as a solar cell.

In the photoelectric conversion elements 10A to 10D, a method of allowing an electron to flow from the photosensitive layer 13 to the conductive support 11 is different depending on presence or absence of the porous layer 12, a kind thereof, and the like. In the photoelectric conversion element 10 of the present invention, electron conduction, in which electrons migrate between the light absorbing agents, occurs. Accordingly, in a case where the porous layer 12 is provided, the porous layer 12 can be formed from an insulating substance other than semiconductors in the related art. In a case where the porous layer 12 is formed from a semiconductor, electron conduction, in which electrons migrate at the inside of semiconductor fine particles of the porous layer 12 or between the semiconductor fine particles, also occurs. On the other hand, in a case where the porous layer 12 is formed from an insulating substance, electron conduction in the porous layer 12 does not occur. In a case where the porous layer 12 is formed from the insulating substance, in a case of using fine particles of an aluminum oxide ($Al_2O_3$) as the fine particles of the insulating substance, a relatively high electromotive force ($V_{OC}$) is obtained.

Even in a case where the blocking layer 14 as the other layer is formed from a conductor or a semiconductor, electron conduction in the blocking layer 14 occurs.

In addition, even in the electron transport layer 15, electron conduction occurs.

The photoelectric conversion element and the solar cell of the present invention are not limited to the preferred aspects, and configurations and the like of the respective aspects may be appropriately combined between the respective aspects in a range not departing from the gist of the present invention.

In the present invention, materials and respective members which are used in the photoelectric conversion element and the solar cell can be prepared by using a typical method except for materials and members which are defined in the present invention. For example, with regard to a perovskite-type solar cell, for example, reference can be made Science 388, 2012, p. 643 to 647, J. Am. Chem. Soc., 2009, 131(17), p. 6050 to 6051, and Science, 338, p. 643 to 647 (2012). In addition, reference can be made to materials and respective members which are used in a dye sensitized

solar cell. With regard to dye sensitized solar cells, for example, reference can be made to JP2001-291534A, US4,927,721A, US4,684,537A, US5,084,365A, US5,350,644A, US5,463,057A, US5,525,440A, JP1995-249790A (JP-H7-249790A), JP2004-220974A, and JP2008-135197A.

Hereinafter, description will be given of preferred aspects of main members and compounds in the photoelectric conversion element and the solar cell of the present invention.

<First Electrode 1>

The first electrode 1 includes the conductive support 11, and at least one of the photosensitive layer 13 or the hole transport layer 16, and functions as an operation electrode in the photoelectric conversion element 10.

According to an aspect, it is preferable that the first electrode 1 includes at least the photosensitive layer 13 between the photosensitive layer 13 and the hole transport layer 16.

In the aspect, it is preferable that the first electrode 1 includes at least one of the porous layer 12, the blocking layer 14, the electron transport layer 15, or the hole transport layer 16 in addition to the conductive support 11 and the photosensitive layer 13 as illustrated in Fig. 1A, and Fig. 2 to Fig. 5.

It is preferable that the first electrode 1 includes at least the blocking layer 14 from the viewpoint of short-circuit prevention, and more preferably the porous layer 12 and the blocking layer 14 from the viewpoints of light absorption efficiency and short-circuit prevention.

In addition, it is preferable that the first electrode 1 includes the electron transport layer 15 and the hole transport layer 16 which are formed from an organic material from the viewpoints of an improvement in productivity of the photoelectric conversion element, thickness reduction, and flexibilization.

- Conductive Support 11 -

The conductive support 11 is not particularly limited as long as the conductive support 11 has conductivity and can support the photosensitive layer 13 and the like. It is preferable that the conductive support 11 has a configuration formed from a conductive material, for example, a metal, or a configuration including the support 11a formed from glass or plastic and the transparent electrode 11b formed on a surface of the support 11a as a conductive film.

Among these, as illustrated in Fig. 1A, and Fig. 2 to Fig. 5, it is more preferable that the conductive support 11 has a configuration in which a conductive metal oxide is applied to the surface of the support 11a formed from glass or plastic to form the transparent electrode 11b. Examples of the support 11a formed from plastic include a transparent polymer film described in Paragraph 0153 of JP2001-291534A. As a material that forms the support 11a, it is possible to use ceramic (for example, refer to JP2005-135902A) and a conductive resin (for example, refer to JP2001-160425A) in addition to glass or plastic. As a metal oxide, a tin oxide (TO) is preferable, and an indium-tin oxide (a tin-doped indium oxide; ITO) or a tin oxide doped with fluorine such as a fluorine-doped tin oxide (FTO) is more preferable. At this time, the amount of the metal oxide applied is preferably 0.1 to 100 g per square meter of a surface area of the support 11a. In a case of using the conductive support 11, it is preferable that light is incident from a support 11a side.

It is preferable that the conductive support 11 is substantially transparent. In the present invention, "substantially transparent" represents that transmittance of light (having a wavelength of 300 to 1200 nm) is 10% or greater, preferably 50% or greater, and more preferably 80% or greater.

The thickness of the support 11a and the conductive support 11 is not particularly limited and is set to an appropriate thickness. For example, the thickness is preferably 0.01 μm to 10 mm, more preferably 0.1 μm to 5 mm, and still preferably 0.3 μm to 4 mm.

In a case of providing the transparent electrode 11b, the film thickness of the transparent electrode 11b is not particularly limited. For example, the film thickness is preferably 0.01 to 30 μm, more preferably 0.03 to 25 μm, and still more preferably 0.05 to 20 μm.

The conductive support 11 or the support 11a may have a light management function on the surface. For example, the conductive support 11 or the support 11a may include an antireflection film formed by alternately laminating a high-refractive-index film and a low-refractive-index oxide film on the surface of the conductive support 11 or the support 11a as described in JP2003-123859A or may have a light guide function as described in JP2002-260746A.

- Blocking Layer 14 -

In the present invention, as in the photoelectric conversion elements 10A to 10C, the blocking layer 14 is preferably provided on the surface of the transparent electrode 11b, that is, between the conductive support 11, and the porous layer 12, the photosensitive layer 13, the hole transport layer 3, or the like.

In the photoelectric conversion element and the solar cell, for example, in a case where the photosensitive layer 13 or the hole transport layer 3, and the transparent electrode 11b and the like are electrically connected to each

other, a reverse current is generated. The blocking layer 14 plays a role of preventing the reverse current. The blocking layer 14 is also referred to as a "short-circuit prevention layer".

The blocking layer 14 may be allowed to function as a stage that carries the light absorbing agent.

The blocking layer 14 may be provided even in a case where the photoelectric conversion element includes the electron transport layer. For example, in a case of the photoelectric conversion element 10D, the blocking layer 14 may be provided between the conductive support 11 and the electron transport layer 15.

The material that forms the blocking layer 14 is not particularly limited as long as the material can perform the above-described function, and it is preferable that the material is a material through which visible light is transmitted, and which has insulating properties with respect to the conductive support 11 (transparent electrode 11b) and the like. Specifically, "material having insulating properties with respect to the conductive support 11 (transparent electrode 11b)" represents a compound (n-type semiconductor compound) having a conduction band energy level that is equal to or higher than a conduction band energy level of a material that forms the conductive support 11 (a metal oxide that forms the transparent electrode 11b) and is lower than a conduction band energy level of a material that constitutes the porous layer 12 or a ground state energy level of the light absorbing agent.

Examples of a material that forms the blocking layer 14 include silicon oxide, magnesium oxide, aluminum oxide, calcium carbonate, cesium carbonate, polyvinyl alcohol, polyurethane, and the like. In addition, the material may be a material that is typically used as a photoelectric conversion material, and examples thereof include titanium oxide, tin oxide, zinc oxide, niobium oxide, tungsten oxide, and the like. Among these, titanium oxide, tin oxide, magnesium oxide, aluminum oxide, and the like are preferred.

It is preferable that the film thickness of the blocking layer 14 is 0.001 to 10 μm, more preferably 0.005 to 1 μm, and still more preferably 0.01 to 0.1 μm.

In the present invention, the film thicknesses of the respective layers can be measured by observing a cross-section of the photoelectric conversion element 10 by using a scanning electron microscope (SEM) and the like.

- Porous Layer 12 -

In the present invention, as in the photoelectric conversion elements 10A and 10B, the porous layer 12 is preferably provided on the transparent electrode 11b. In a case where the photoelectric conversion element 10 includes the blocking layer 14, the porous layer 12 is preferably formed on the blocking layer 14.

The porous layer 12 is a layer that functions as a stage that carries the photosensitive layer 13 on the surface. In a solar cell, so as to increase the light absorption efficiency, it is preferable to increase a surface area of at least a portion that receives light such as solar light, and it is preferable to increase the surface area of the porous layer 12 as a whole.

It is preferable that the porous layer 12 is a fine particle layer that includes pores and is formed through deposition or close contact of fine particles of a material that forms the porous layer 12. The porous layer 12 may be a fine particle layer that is formed through deposition of two or more kinds of fine particles. In a case where the porous layer 12 is a fine particle layer that includes pores, it is possible to increase the amount (adsorption amount) of the carried light absorbing agent.

It is preferable to increase the surface area of individual fine particles which constitute the porous layer 12 so as to increase the surface area of the porous layer 12. In the present invention, in a state in which the fine particles are applied to the conductive support 11 and the like, it is preferable that the surface area of the fine particles which form the porous layer 12 is 10 or more times a projected area, and more preferably 100 or more times the projected area. The upper limit thereof is not particularly limited. Typically, the upper limit is approximately 5000 times the projected area. With regard to a particle size of the fine particles which form the porous layer 12, an average particle size, which uses a diameter when converting the projected area into a circle, is preferably 0.001 to 1 μm as primary particles. In a case where the porous layer 12 is formed by using a dispersion of fine particles, the average particle size of the fine particles is preferably 0.01 to 100 μm in terms of an average particle size of the dispersion.

For the material that forms the porous layer 12, there is no particular limitation with respect to conductivity. The material may be an insulating substance (insulating material), a conductive material, or a semiconductor (semi-conductive material).

As the material that forms the porous layer 12, it is possible to use, for example, chalcogenides (for example, an oxide, a sulfide, a selenide, and the like) of metals, compounds having a perovskite-type crystal structure (excluding a perovskite compound that uses a light absorbing agent), oxides of silicon (for example, silicon dioxide, and zeolite), or carbon nanotubes (including carbon nanowires, carbon nanorods, and the like).

The chalcogenides of a metal are not particularly limited, and preferred examples thereof include respective oxides of titanium, tin, zinc, tungsten, zirconium, hafnium, strontium, indium, cerium, yttrium, lanthanum, vanadium, niobium, aluminum, and tantalum, cadmium sulfide, cadmium selenide, and the like. Examples of the crystal structure of the chalcogenides of metals include an anatase-type crystal structure, a brookite-type crystal structure, and a rutile-type crystal structure, and the anatase-type crystal structure and the brookite-type crystal structure are preferable.

The compound having a perovskite-type crystal structure is not particularly limited, and examples thereof include a transition metal oxide and the like. Examples of the transition metal oxide include strontium titanate, calcium titanate, barium titanate, zinc titanate, barium zirconate, barium stannate, zinc zirconate, strontium zirconate, strontium tantalate, potassium niobate, bismuth ferrate, barium strontium titanate, lanthanum barium titanate, calcium titanate, sodium titanate, and bismuth titanate. Among these, strontium titanate, calcium titanate, and the like are preferable.

The carbon nanotubes have a shape obtained by rounding off a carbon film (graphene sheet) into a tubular shape. The carbon nanotubes are classified into a single-walled carbon nanotube (SWCNT) obtained by winding one graphene sheet in a cylindrical shape, a double-walled carbon nanotube (DWCNT) obtained by winding two graphene sheets in a concentric shape, and a multi-walled carbon nanotube (MWCNT) obtained by winding a plurality of graphene sheets in a concentric shape. As the porous layer 12, any carbon nanotubes can be used without any particular limitation.

Among these, as the material that forms the porous layer 12, an oxide of titanium, tin, zinc, zirconium, aluminum, or silicon, or a carbon nanotube is preferable, and titanium oxide or aluminum oxide is more preferable.

The porous layer 12 may be formed from at least one kind of the chalcogenides of metals, the compound having a perovskite-type crystal structure, the oxide of silicon, or the carbon nanotube, or may be formed from a plurality of kinds thereof.

The film thickness of the porous layer 12 is not particularly limited. The thickness is typically in a range of 0.05 to 100 $\mu$m, and preferably in a range of 0.1 to 100 $\mu$m. In a case of being used as a solar cell, the film thickness is preferably 0.1 to 50 $\mu$m, and more preferably 0.2 to 30 $\mu$m.

- Electron Transport Layer 15 -

In the present invention, as in the photoelectric conversion element 10D, the electron transport layer 15 is preferably provided on the surface of the transparent electrode 11b.

The electron transport layer 15 has a function of transporting electrons, which are generated in the photosensitive layer 13, to the conductive support 11. The electron transport layer 15 is formed from an electron transporting material capable of exhibiting the above-described function. The electron transporting material is not particularly limited, and an organic material (organic electron transporting material) is preferable. Examples of the organic electron transporting material include fullerene compounds such as [6,6]-phenyl-C61-butyric acid methyl ester ($PC_{61}BM$), perylene compounds such as perylene tetracarboxylic diimide (PTCDI), low-molecular-weight compounds such as tetracyano-quinodimethane (TCNQ), high-molecular-weight compounds, and the like.

Although not particularly limited, it is preferable that the film thickness of the electron transport layer 15 is 0.001 to 10 $\mu$m, and more preferably 0.01 to 1 $\mu$m.

- Hole Transport Layer 16 -

In the present invention, as in the photoelectric conversion element 10E, the hole transport layer 16 may be provided on the surface of the transparent electrode 11b.

The hole transport layer 16 is the same as the hole transport layer 3 to be described later except for a different formation position.

- Photosensitive layer (Light Absorbing Layer) 13 -

The photosensitive layer 13 is preferably provided on the surface (including an inner surface of a concave portion in a case where a surface on which the photosensitive layer 13 is provided is uneven) of each of the porous layer 12 (in the photoelectric conversion elements 10A and 10B), the blocking layer 14 (in the photoelectric conversion element 10C), the electron transport layer 15 (in the photoelectric conversion element 10D), and the hole transport layer 16 (in the photoelectric conversion element 10E).

In the present invention, the photosensitive layer 13 may contain at least one kind of perovskite compound to be described later, or two or more kinds of perovskite compounds as the light absorbing agent. In addition, the photosensitive layer 13 may include a light absorbing agent other than the perovskite compound in combination with the perovskite compound as a light absorbing agent. Examples of the light absorbing agent other than the perovskite compound include a metal complex dye, and an organic dye. At this time, a ratio between the perovskite compound and the light absorbing agent other than the perovskite compound is not particularly limited.

The photosensitive layer 13 may be a monolayer or a laminated layer of two or more layers. In a case where the photosensitive layer 13 has the laminated layer structure of two or more layers, the laminated layer structure may be a laminated layer structure obtained by laminating layers formed from light absorbing agents different from each other, or a laminated layer structure including an interlayer including a hole transporting material between a photosensitive

layer and a photosensitive layer.

The aspect in which the photosensitive layer 13 is provided on the conductive support 11 is as described above. The photosensitive layer 13 is preferably provided on a surface of each of the layers in order for an excited electron to flow to the conductive support 11 or the second electrode 2. At this time, the photosensitive layer 13 may be provided on the entirety or a part of the surface of each of the layers.

The film thickness of the photosensitive layer 13 is appropriately set in correspondence with an aspect in which the photosensitive layer 13 is provided on the conductive support 11, and is not particularly limited. For example, the film thickness is preferably 0.001 to 100 μm, more preferably 0.01 to 10 μm, and still more preferably 0.01 to 5 μm.

In a case where the porous layer 12 is provided, a total film thickness including the film thickness of the porous layer 12 is preferably 0.01 μm or greater, more preferably 0.05 μm or greater, still more preferably 0.1 μm or greater, and still more preferably 0.3 μm or greater. In addition, the total film thickness is preferably 100 μm or less, more preferably 50 μm or less, and still more preferably 30 μm or less. The total film thickness may be set to a range in which the above-described values are appropriately combined. Here, as illustrated in Fig. 1A and Fig. 1B, in a case where the photosensitive layer 13 has a thin film shape, the film thickness of the photosensitive layer 13 represents a distance between an interface with the porous layer 12, and an interface with the hole transport layer 3 to be described later along a direction that is perpendicular to the surface of the porous layer 12.

In the photoelectric conversion element 10, a total film thickness of the porous layer 12, the photosensitive layer 13, and the hole transport layer 3 is not particularly limited. For example, the total thickness is preferably 0.01 μm or greater, more preferably 0.05 μm or greater, still more preferably 0.1 μm or greater, and still more preferably 0.5 μm or greater. In addition, the total film thickness is preferably 200 μm or less, more preferably 50 μm or less, still more preferably 30 μm or less, and still more preferably 5 μm or less. The total film thickness may be set to a range in which the above-described values are appropriately combined.

In the present invention, in a case where the photosensitive layer is provided in a thick film shape (in the photosensitive layer 13B and 13C), the light absorbing agent that is included in the photosensitive layer may function as a hole transporting material.

The amount of the perovskite compound used may be set to an amount capable of covering at least a part of a surface of the first electrode 1, and preferably an amount capable of covering the entirety of the surface.

[Light Absorbing Agent of Photosensitive Layer]

The photosensitive layer 13 contains at least one kind of perovskite compound (perovskite-type light absorbing agent) having a perovskite-type crystal structure that includes "an element of Group 1 in the periodic table or a cationic organic group A", a metal atom M other than elements of Group 1 in the periodic table", and "an anionic atom or atomic group X" as the light absorbing agent.

In the perovskite compound, the element of Group 1 in the periodic table or the cationic organic group A, the metal atom M, and the anionic atom or atomic group X exists as individual constituent ions of a cation (for convenience, may be referred to as "cation A"), a metal cation (for convenience, may be referred to as "cation M"), and an anion (for convenience, may be referred to as "anion X") in the perovskite-type crystal structure.

In the present invention, the cationic organic group represents an organic group having a property of becoming a cation in the perovskite-type crystal structure, and the anionic atom or atomic group represents an atom or atomic group that has a property of becoming an anion in the perovskite-type crystal structure.

In the perovskite compound that is used in the present invention, the cation A represents a cation of an element of Group 1 in the periodic table or an organic cation that is composed of a cationic organic group A. The cation A is preferably an organic cation.

The cation of an element of Group 1 in the periodic table is not particularly limited, and examples thereof include cations ($Li^+$, $Na^+$, $K^+$, and $Cs^+$) of individual elements of lithium (Li), sodium (Na), potassium (K), and cesium (Cs), and the cation ($Cs^+$) of cesium is more preferable.

The organic cation is not particularly limited as long as the organic cation is a cation of an organic group having the above-described property, but an organic cation of a cationic organic group represented by the following Formula (1) is more preferable.

Formula (1): $R^{1a}\text{-}NH_3^+$

In Formula (1), $R^{1a}$ represents a substituent. $R^{1a}$ is not particularly limited as long as $R^{1a}$ is an organic group, but an alkyl group, a cycloalkyl group, an alkenyl group, an alkynyl group, an aryl group, a heteroaryl group, or a group represented by the following Formula (2) is preferable. Among these, the alkyl group and a group represented by the following Formula (2) are more preferable.

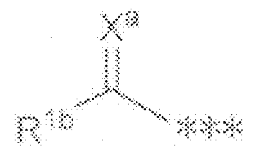

Formula (2)

In Formula (2), $X^a$ represents $NR^{1c}$, an oxygen atom, or a sulfur atom. $R^{1b}$ and $R^{1c}$ each independently represent a hydrogen atom or a substituent. *** represents bonding with a nitrogen atom in Formula (1).

In the present invention, as the organic cation of the cationic organic group A, an organic ammonium cation ($R^{1a}$-$NH_3^+$) composed of an ammonium cationic organic group A obtained through bonding between $R^{1a}$ and $NH_3$ in Formula (1) is preferable. In a case where the organic ammonium cation can employ a resonance structure, the organic cation further includes a cation having the resonance structure in addition to the organic ammonium cation. For example, in a case where $X^a$ is NH ($R^{1c}$ is a hydrogen atom) in a group represented by Formula (2), the organic cation also includes an organic amidinium cation that is one of a resonance structure of the organic ammonium cation in addition to the organic ammonium cation of the ammonium cationic organic group obtained through bonding between the group represented by Formula (2) and $NH_3$. Examples of the organic amidinium cation composed of the amidinium cationic organic group include a cation represented by the following Formula ($A^{am}$). In this specification, the cation represented by the following Formula ($A^{am}$) may be noted as "$R^{1b}C(=NH)-NH_3^+$" for convenience.

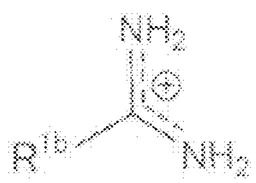

Formula ($A^{am}$)

The alkyl group as $R^{1a}$ in Formula (1) is preferably an alkyl group having 1 to 18 carbon atoms, more preferably an alkyl group having 1 to 6 carbon atoms, and still more preferably an alkyl group having 1 to 3 carbon atoms. Examples of the alkyl group include methyl, ethyl, propyl, isopropyl, butyl, tert-butyl, pentyl, hexyl, decyl, and the like.

The cycloalkyl group as $R^{1a}$ is preferably a cycloalkyl group having 3 to 8 carbon atoms, and examples thereof include cyclopropyl, cyclopentyl, cyclohexyl, and the like.

The alkenyl group as $R^{1a}$ is preferably an alkenyl group having 2 to 18 carbon atoms, and more preferably an alkenyl group having 2 to 6 carbon atoms. Examples of the alkenyl group include vinyl, allyl, butenyl, hexenyl, and the like.

The alkynyl group as $R^{1a}$ is preferably an alkynyl group having 2 to 18 carbon atoms, and more preferably an alkynyl group having 2 to 4 carbon atoms. Examples of the alkynyl group include ethynyl, butynyl, hexynyl, and the like.

The aryl group as $R^{1a}$ is preferably an aryl group having 6 to 14 carbon atoms, and more preferably an aryl group having 6 to 12 carbon atoms, and examples thereof include phenyl.

The heteroaryl group as $R^{1a}$ includes a group composed of an aromatic hetero ring alone, and a group composed of a fused hetero ring obtained through condensing of another ring, for example, an aromatic ring, an aliphatic ring, or a hetero ring with the aromatic hetero ring.

As the ring-constituting hetero atom that constitutes the aromatic hetero ring, a nitrogen atom, an oxygen atom, or a sulfur atom is preferable. In addition, with regard to the number of ring members of the aromatic hetero ring, a 3- to 8-membered ring is preferable, and a 5- or 6-membered ring is more preferable. In regard to the number of carbon atoms of the aromatic hetero ring, 0 to 24 carbon atoms are preferable, and 1 to 18 carbon atoms are more preferable.

Examples of the 5-membered aromatic hetero ring and the fused hetero ring including the 5-membered aromatic hetero ring include respective cyclic groups of a pyrrole ring, an imidazole ring, a pyrazole ring, an oxazole ring, a thiazole ring, a triazole ring, a furan ring, a thiophene ring, a benzimidazole ring, a benzoxazole ring, a benzothiazole ring, an indoline ring, and an indazole ring. In addition, examples of the 6-membered aromatic hetero ring and the fused hetero ring including the 6-membered aromatic hetero ring include respective cyclic groups of a pyridine ring, a pyrimidine ring, a pyrazine ring, a triazine ring, a quinoline ring, and a quinazoline ring.

In the group represented by Formula (2), $X^a$ represents $NR^{1c}$, an oxygen atom, or a sulfur atom, and $NR^{1c}$ is preferable as $X^a$. Here, $R^{1c}$ represents a hydrogen atom or a substituent. $R^{1c}$ is preferably a hydrogen atom, an alkyl group, a cycloalkyl group, an alkenyl group, an alkynyl group, an aryl group, or a heteroaryl group, and more preferably a hydrogen atom.

$R^{1b}$ represents a hydrogen atom or a substituent, and is preferably a hydrogen atom. Examples of the substituent that can be employed as $R^{1b}$ include an amino group, an alkyl group, a cycloalkyl group, an alkenyl group, an alkynyl group, an aryl group, a heteroaryl group.

An alkyl group, a cycloalkyl group, an alkenyl group, an alkynyl group, an aryl group, and a heteroaryl group that can be respectively employed as $R^{1b}$ and $R^{1c}$ are the same as the respective groups of $R^{1a}$, and preferred examples thereof are the same as described above.

Examples of the group represented by Formula (2) include a (thio)acyl group, a (thio)carbamoyl group, an imidoyl group, and an amidino group.

Examples of the (thio)acyl group include an acyl group and a thioacyl group. The acyl group is preferably an acyl group having a total of 1 to 7 carbon atoms, and examples thereof include formyl, acetyl, propionyl, hexanoyl, and the like. The thioacyl group is preferably a thioacyl group having a total of 1 to 7 carbon atoms, and examples thereof include thioformyl, thioacetyl, thiopropionyl, and the like.

Examples of the (thio)carbamoyl group include a carbamoyl group and a thiocarbamoyl group ($H_2NC(=S)-$).

The imidoyl group is a group represented by $R^{1b}-C(=NR^{1c})-$, and it is preferable that $R^{1b}$ and $R^{1c}$ are respectively a hydrogen atom and an alkyl group. More preferably, the alkyl group is the same as the alkyl group as $R^{1a}$. Examples thereof include formimidoyl, acetoimidoyl, propionimidoyl ($CH_3CH_2C(=NH)-$), and the like. Among these, formimidoyl is preferable.

The amidino group as the group represented by Formula (2) has a structure in which $R^{1b}$ of the imidoyl group is an amino group and $R^{1c}$ is a hydrogen atom.

The entirety of the alkyl group, the cycloalkyl group, the alkenyl group, the alkynyl group, the aryl group, the heteroaryl group, and the group represented by Formula (2), which can be employed as $R^{1a}$, may have a substituent. The substituent $W^P$, which $R^{1a}$ may have, is not particularly limited, and examples thereof include an alkyl group, a cycloalkyl group, an alkenyl group, an alkynyl group, an aryl group, a heterocyclic group, an alkoxy group, an alkylthio group, an amino group, an alkylamino group, an arylamino group, an acyl group, an alkylcarbonyloxy group, an aryloxy group, an alkoxycarbonyl group, an aryloxycarbonyl group, an acylamino group, a sulfonamide group, a carbamoyl group, a sulfamoyl group, a halogen atom, a cyano group, a hydroxy group, a mercapto group, and a carboxy group. The substituent, which $R^{1a}$ may have, may be additionally substituted with a substituent.

In the perovskite compound that is used in the present invention, the metal cation M is not particularly limited as long as the metal cation M is a cation of a metal atom other than elements of Group 1 in the periodic table and is a cation of a metal atom that can employ the perovskite-type crystal structure. Examples of the metal atom include metal atoms such as calcium (Ca), strontium (Sr), cadmium (Cd), copper (Cu), nickel (Ni), manganese (Mn), iron (Fe), cobalt (Co), palladium (Pd), germanium (Ge), tin (Sn), lead (Pb), ytterbium (Yb), europium (Eu), indium (In), titanium (Ti), bismuth (Bi), and thallium (Tl). Among these, as the metal atom M, a Pb atom or a Sn atom is more preferable. M may be one kind of metal atom, or two or more kinds of metal atoms. In a case where M includes two or more kinds of metal atoms, two kinds including the Pb atom and the Sn atom are preferable. A ratio of the metal atoms at this time is not particularly limited.

In the perovskite compound that is used in the present invention, the anion X represents an anion of an anionic atom or atomic group X. Preferred examples of the anion include anions of halogen atoms, and anions of individual atomic groups of $NCS^-$, $NCO^-$, $CH_3COO^-$, and $HCOO^-$. Among these, the anions of halogen atoms are more preferable. Examples of the halogen atoms include a fluorine atom, a chlorine atom, a bromine atom, an iodine atom, and the like.

The anion X may be an anion of one kind of anionic atom or atomic group, or anions of two or more kinds of anionic atoms or atomic groups. In a case where the anion X is an anion of one kind of anionic atom or atomic group, an anion of an iodine atom is preferable. On the other hand, in a case where the anion X includes anions of two or more kinds of anionic atoms or atomic groups, anions of two kinds of halogen atoms, particularly, an anion of a chlorine atom and an anion of an iodine atom are preferable. A ratio between two or more kinds of anions is not particularly limited.

As the perovskite compound that is used in the present invention, a perovskite compound, which has a perovskite-type crystal structure including the above-described constituent ions and is represented by the following Formula (I), is preferable.

Formula (I): $A_aM_mX_x$

In Formula (I), A represents an element of Group 1 in the periodic table or a cationic organic group. M represents a metal atom other than elements of Group 1 in the periodic table. X represents an anionic atom or atomic group.
a represents 1 or 2, m represents 1, and a, m, and x satisfy a relationship of $a+2m = x$.

In Formula (I), the element of Group 1 in the periodic table or the cationic organic group A forms the cation A of the perovskite-type crystal structure. Accordingly, there is no particular limitation as long as the element of Group 1 in the periodic table and the cationic organic group A are elements or groups which become the cation A and can constitute the perovskite-type crystal structure. The element of Group 1 in the periodic table or the cationic organic group

A is the same as the element of Group 1 in the periodic table or the cationic organic group which is described in the cation A, and preferred examples thereof are the same as described above.

The metal atom M is a metal atom that forms the metal cation M of the perovskite-type crystal structure. Accordingly, the metal atom M is not particularly limited as long as the metal atom M is an atom other than elements of Group 1 in the periodic table, becomes the metal cation M, and constitutes the perovskite-type crystal structure. The metal atom M is the same as the metal atom that is described in the metal cation M, and preferred examples thereof are the same as described above.

The anionic atom or atomic group X forms the anion X of the perovskite-type crystal structure. Accordingly, the anionic atom or atomic group X is not particularly limited as long as the anionic atom or atomic group X is an atom or atomic group that becomes the anion X and can constitute the perovskite-type crystal structure. The anionic atom or atomic group X is the same as the anionic atom or atomic group which is described in the anion X, and preferred examples thereof are the same as described above.

The perovskite compound represented by Formula (I) is a perovskite compound represented by the following Formula (I-1) in a case where a is 1, or a perovskite compound represented by the following Formula (I-2) in a case where a is 2.

Formula (I-1): $AMX_3$

Formula (I-2): $A_2MX_4$

In Formula (I-1) and Formula (I-2), A represents an element of Group 1 in the periodic table or a cationic organic group. A is the same as A in Formula (I), and preferred examples thereof are the same as described above.

M represents a metal atom other than elements of Group 1 in the periodic table. M is the same as M in Formula (I), and preferred examples thereof are the same as described above.

X represents an anionic atom or atomic group. X is the same as X in Formula (I), and preferred examples thereof are the same as described above.

The perovskite compound that is used in the present invention may be any one of the compound represented by Formula (I-1) and the compound represented by Formula (I-2), or a mixture thereof. Accordingly, in the present invention, at least one kind of the perovskite compound may exist as the light absorbing agent, and there is no need for clear and strict distinction on that the perovskite compound is which compound by using a composition formula, a molecular formula, a crystal structure, and the like.

Hereinafter, specific examples of the perovskite compound that can be used in the present invention will be exemplified, but the present invention is not limited to the specific examples. In the following description, the perovskite compound is classified into the compound represented by Formula (I-1) and the compound represented by Formula (I-2). However, even the compound exemplified as the compound represented by Formula (I-1) may be the compound represented by Formula (I-2) in accordance with synthesis conditions, or may be a mixture of the compound represented by Formula (I-1) and the compound represented by Formula (I-2). Similarly, even the compound exemplified as the compound represented by Formula (I-2) may be the compound represented by Formula (I-1), or may be a mixture of the compound represented by Formula (I-1) and the compound represented by Formula (I-2).

Specific examples of the compound represented by Formula (I-1) include $CH_3NH_3PbCl_3$, $CH_3NH_3PbBr_3$, $CH_3NH_3PbI_3$, $CH_3NH_3PbBrI_2$, $CH_3NH_3PbBr_2I$, $CH_3NH_3SnBr_3$, $CH_3NH_3SnI_3$, and $CH(=NH)NH_3PbI_3$.

Specific examples of the compound represented by Formula (1-2) include $(C_2H_5NH_3)_2PbI_4$, $(CH_2=CHNH_3)_2PbI_4$, $(CH=CNH_3)_2PbI_4$, $(n\text{-}C_3H_7NH_3)_2PbI_4$, $(n\text{-}C_4H_9NH_3)_2PbI_4$, $(C_{10}H_{21}NH_3)_2PbI_4$, $(C_6H_5NH_3)_2PbI_4$, $(C_6H_6CH_2CH_2NH_3)_2PbI_4$, $(C_6H_3F_2NH_3)_2PbI_4$, $(C_6F_5NH_3)_2PbI_4$, $(C_4H_3SNH_3)_2PbI_4$. Here, $C_4H_3SNH_3$ in $(C_4H_3SNH_3)_2PbI_4$ represents aminothiophene.

The perovskite compound can be synthesized from a compound represented by the following Formula (II) and a compound represented by the following Formula (III).

Formula (II): $AX$

Formula (III): $MX_2$

In Formula (II), A represents an element of Group 1 in the periodic table, or a cationic organic group. A is the same as A in Formula (I), and preferred examples thereof are the same as described above. In Formula (II), X represents an anionic atom or atomic group. X is the same as X in Formula (I), and preferred examples thereof are the same as described above.

In Formula (III), M represents a metal atom other than elements of Group 1 in the periodic table. M is the same as M in Formula (I), and preferred examples thereof are the same as described above. In Formula (III), X represents an anionic atom or atomic group. X is the same as X in Formula (I), and preferred examples thereof are the same as described above.

Examples of a method of synthesizing the perovskite compound include methods which are described in Akihiro Kojima, Kenjiro Teshima, Yasuo Shirai, and Tsutomu Miyasaka, "Organometal Halide Perovskites as Visible-Light Sensitizers for Photovoltaic Cells", J. Am. Chem. Soc., 2009, 131(17), 6050 to 6051, and the like.

The amount of the light absorbing agent used is preferably set to an amount capable of covering at least a part of the surface of the first electrode 1, and more preferably an amount capable of covering the entirety of the surface.

The amount of the perovskite compound contained in the photosensitive layer 13 is typically 1% to 100% by mass.

<Hole Transport Layer 3>

In the photoelectric conversion element of the present invention, as in the photoelectric conversion elements 10A to 10D, an aspect in which the hole transport layer 3 is provided between the first electrode 1 and the second electrode 2 is also preferable. In the aspect, it is preferable that the hole transport layer 3 is in contact with (laminated on) the photosensitive layer 13. The hole transport layer 3 is preferably provided between the photosensitive layer 13 of the first electrode 1 and the second electrode 2.

The hole transport layer 3 includes a function of supplementing electrons to an oxidized substance of the light absorbing agent, and is preferably a solid-shaped layer (solid hole transport layer).

The hole transport layer 3 contains the compound Q (hereinafter, also referred to as "hole transporting material of the present invention" or "compound of the present invention") having at least one structure M represented by Formula 1 as a hole transporting material.

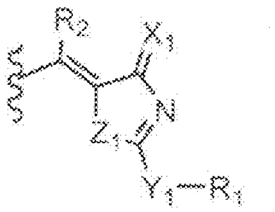

Formula 1

In Formula 1, $Z_1$ represents a sulfur atom, a selenium atom, or an oxygen atom, $Y_1$ represents NR, a sulfur atom, a selenium atom, or an oxygen atom, $X_1$ represents NR, a sulfur atom, an oxygen atom, a selenium atom, or $CR_3R_4$, $R_1$ represents a substituent, $R_2$, $R_3$, $R_4$, and R each independently represent a hydrogen atom or a substituent, and a wavy line represents a binding site with a remainder of the compound Q.

The compound Q having the structure M represented by Formula 1 is more hydrophobic compared to other compounds of comparable size containing a hetero atom, and it is presumed that a case of using the compound Q as the hole transporting material is contributable to suppressing the moisture resistance variation. In addition, the compound Q having the structure M has high affinity for the perovskite compound, and it is presumed that a case of using the compound Q in the photoelectric conversion element containing the perovskite compound as the light absorbing agent is contributable to further improvement in the effect of suppressing the moisture resistance variation.

In one aspect, the compound Q preferably has 1 to 8 structures M represented by Formula 1, more preferably 1 to 4 structures M, and even more preferably 2 structures M.

In a case where the compound Q has two or more of the structures M, the two or more structures M may be the same as or different from each other. In one aspect of the present invention, in the case where the compound Q has two or more of the structures M, all the structures M are preferably the same as each other.

Hereinafter, detailed description will be given of Formula 1.

As described above, $Z_1$ represents a sulfur atom, a selenium atom, or an oxygen atom, and it is preferable that $Z_1$ is a sulfur atom in one aspect.

As described above, $Y_1$ represents NR, a sulfur atom, a selenium atom, or an oxygen atom.

NR in R represents a hydrogen atom or a substituent. Examples of the substituent include an alkyl group having 1 to 30 carbon atoms, a cycloalkyl group having 3 to 30 carbon atoms, an alkenyl group having 2 to 30 carbon atoms, an alkynyl group having 2 to 30 carbon atoms, an aromatic group having 4 to 30 carbon atoms, an aromatic heterocyclic group having 4 to 30 carbon atoms, an aliphatic heterocyclic group having 3 to 30 carbon atoms, and the like, and an alkyl group having 1 to 30 carbon atoms is preferable.

$Y_1$ is preferably a sulfur atom in one aspect.

As described above, $X_1$ represents NR, a sulfur atom, an oxygen atom, a selenium atom, or $CR_3R_4$.

R in NR represents a hydrogen atom or a substituent, and examples of the substituent include an alkyl group, a cycloalkyl group, an alkenyl group, an alkynyl group, an aromatic group, an aromatic heterocyclic group, and the like.

In addition, $R_3$ and $R_4$ in $CR_3R_4$ each independently represent a hydrogen atom or a substituent, and examples of the substituent include an alkyl group, a cycloalkyl group, an alkenyl group, an alkynyl group, an aromatic group, an aromatic heterocyclic group, an alkoxy group, an alkylthio group, an amino group, an alkylamino group, an arylamino group, an acyl group, an alkylcarbonyloxy group, an aryloxy group, an alkoxycarbonyl group, an aryloxycarbonyl group, an acylamino group, a sulfonamide group, a carbamoyl group, a sulfamoyl group, a halogen atom, a cyano group, a hydroxy group, a mercapto group, a silyl group, a carboxy group, and the like, and a cyano group and a carboxy group are preferable.

$X_1$ is preferably an oxygen atom in one aspect.

As described above, $R_2$ represents a hydrogen atom or a substituent. Examples of the substituent include an alkyl group, a cycloalkyl group, an alkenyl group, an alkynyl group, an aromatic group, an aromatic heterocyclic group, an alkoxy group, an alkylthio group, an amino group, an alkylamino group, an arylamino group, an acyl group, an alkylcarbonyloxy group, an aryloxy group, an alkoxycarbonyl group, an aryloxycarbonyl group, an acylamino group, a sulfonamide group, a carbamoyl group, a sulfamoyl group, a halogen atom, a cyano group, a hydroxy group, a mercapto group, a silyl group, a carboxy group, and the like.

As described above, $R_1$ represents a substituent. Examples of the substituent include an alkyl group having 1 to 30 carbon atoms, a cycloalkyl group having 3 to 30 carbon atoms, an alkenyl group having 2 to 30 carbon atoms, an alkynyl group having 2 to 30 carbon atoms, an aromatic group having 4 to 30 carbon atoms, an aromatic heterocyclic group having 4 to 30 carbon atoms, an aliphatic heterocyclic group having 3 to 30 carbon atoms, and the like, and an alkyl group having 1 to 30 carbon atoms is preferable.

Specific examples of the structure M represented by Formula 1 include the following structure.

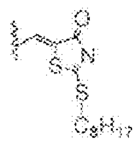 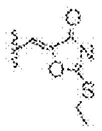 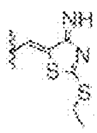 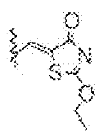 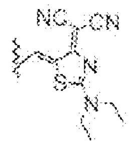

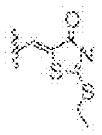 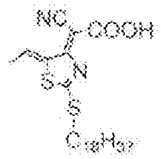 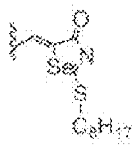 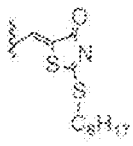 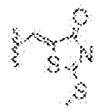

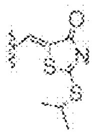 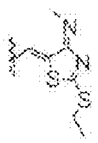

The compound Q is preferably a compound represented by Formula 2 in one aspect.

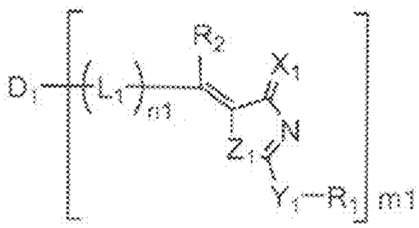

Formula 2

In Formula 2, $Z_1$ represents a sulfur atom, a selenium atom, or an oxygen atom, $Y_1$ represents NR, a sulfur atom, a selenium atom, or an oxygen atom, $X_1$ represents NR, a sulfur atom, an oxygen atom, a selenium atom, or $CR_3R_4$, $R_1$ represents a substituent, $R_2$, $R_3$, $R_4$, and R each independently represent a hydrogen atom or a substituent, and $L_1$ represents a linking group.

$D_1$ represents an organic group or a nitrogen atom. The organic group represented by $D_1$ may have another

substituent in Formula 2 that is different from a substituent represented by Formula 2a, and the nitrogen atom may have another substituent that is different from a substituent represented by Formula 2a or a hydrogen atom. $Z_1$, $Y_1$, $X_1$, $R_1$, $R_2$, $L_1$, n1, and m1 in Formula 2a are the same as $Z_1$, $Y_1$, $X_1$, $R_1$, $R_2$, $L_1$, n1, and m1 in Formula 2.

n1 represents an integer of 0 to 11. In a case where n1 is an integer of 2 or more, a plurality of $L_1$'s may be the same as or different from each other.

In a case where $D_1$ is an organic group, m1 represents an integer of 1 to 4, and in a case where $D_1$ is a nitrogen atom, m1 represents an integer of 1 to 3, provided that in the case where $D_1$ is a nitrogen atom, m1 is an integer of 1 to 3. In a case where m1 is an integer of 2 or more, a plurality of $Z_1$'s, $Y_1$'s, $X_1$'s, $R_1$'s, and $R_2$'s may be the same as or different from each other, respectively.

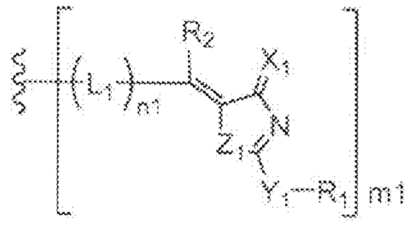

Formula 2a

$Z_1$, $Y_1$, $X_1$, $R_1$, and $R_2$ in Formula 2 are the same as $Z_1$, $Y_1$, $X_1$, $R_1$, and $R_2$ in Formula 1 described above.

$L_1$ represents a linking group as described above, and in the case where n1 is an integer of 2 or more, a plurality of $L_1$'s may be the same as or different from each other. n1 is preferably an integer of 0 to 11, and more preferably an integer of 1 to 5.

Examples of the linking group represented by $L_1$ include an alkenylene group, an alkynylene group, an arylene group, a heteroarylene group, and the like.

The alkenylene group as $L_1$ is preferably an alkenylene group having 2 to 30 carbon atoms, and examples thereof include ethenylene, 1-propenylene, 2-butenylene, 1-butenylene, 3-methyl-2-pentenylene, hexenylene, decenylene, and the like. Furthermore, in an alkenylene group, sis-trans isomerism is not particularly limited.

The cycloalkenylene group as $L_1$ is preferably a cycloalkenylene group having 5 to 25 carbon atoms, and examples thereof include cyclopentenylene, cyclohexenylene, cyclohexadienylene, cyclooctenylene, cyclooctadienylene, and the like.

An alkynylene group as $L_1$ is preferably an alkynylene having 2 to 6 carbon atoms, and examples thereof include an ethynylene and the like.

The arylene group as $L_1$ is preferably an arylene group having 6 to 14 carbon atoms, and specific examples thereof include a phenylene group, a naphthylene group, an anthracenylene group, and the like.

In a heteroarylene group as $L_1$, as the ring-constituting hetero atom, a nitrogen atom, an oxygen atom, or a sulfur atom is preferable, and a heteroarylene group containing at least one selected from these atoms as the ring-constituting hetero atom is preferable. In addition, the heteroarylene group preferably has 0 to 24 carbon atoms and more preferably has 1 to 18 carbon atoms, and with regard to the number of ring members, a 3- to 8-membered ring is preferable, and a 5- or 6-membered ring is more preferable.

Examples of the heteroarylene group of the 5-membered ring include a pyrrolylene group, an imidazolylene group, a pyrazolylene group, an oxazolylene group, a thiazolylene group, a triazolylene group, a furanylene group, a thiophenylene group, and the like. In addition, examples of the 6-membered aromatic hetero ring and the fused hetero ring including the 6-membered aromatic hetero ring include a pyridinylene group, a pyrimidinylene group, a pyrazinylene group, a triazinylene group, and the like.

In one aspect of the present invention, at least one $L_1$ is preferably a heteroarylene group, at least one $L_1$ is more preferably a thiophenylene group or a thiazolylene group, and at least one $L_1$ is even more preferably a thiophenylene group.

It is preferable that the linking group represented by $L_1$ further has a substituent. The substituent is not particularly limited, and examples thereof include an alkyl group, a cycloalkyl group, an alkenyl group, an alkynyl group, an aryl group, a heterocyclic group, an alkoxy group, an alkylthio group, an amino group, an alkylamino group, an arylamino group, an acyl group, an alkylcarbonyloxy group, an aryloxy group, an alkoxycarbonyl group, an aryloxycarbonyl group, an acylamino group, a sulfonamide group, a carbamoyl group, a sulfamoyl group, a halogen atom, a cyano group, a hydroxy group, a mercapto group, a silyl group, and a carboxy group.

As described above, $D_1$ represents an organic group or a nitrogen atom.

The organic group represented by $D_1$ represents a group containing at least one carbon atom, and examples thereof include an alkenyl group, a cycloalkenyl group, an alkynyl group, an aromatic ring group, an aliphatic heterocyclic group, an aromatic heterocyclic group, a fused ring group containing at least one aromatic ring, a fused ring group

containing at least one aromatic hetero ring, and the like.

An alkenyl group represented by $D_1$ preferably has 2 to 30 carbon atoms, and examples thereof include ethenyl, 1-propenyl, 2-butenyl, 1-butenyl, 3-methyl-2-pentenyl, hexenyl, decenyl, and the like. Furthermore, in the alkenyl group, sis-trans isomerism is not particularly limited.

The cycloalkenyl group preferably has 5 to 30 carbon atoms, and examples thereof include cyclopentenyl, cyclohexenyl, cyclohexadienyl, cyclooctenyl, cyclooctadienyl, and the like.

The alkynyl group preferably has 2 to 6 carbon atoms, and examples thereof include ethynyl and the like.

The aromatic ring group is preferably, for example, an aromatic ring group having 6 to 30 carbon atoms, and specific examples thereof include a benzene ring, a naphthalene ring, an anthracene ring, a pentacene ring, and the like.

The aliphatic heterocyclic group is preferably, for example, an aliphatic heterocyclic group having 2 to 30 carbon atoms, and specific examples thereof include a morpholine ring, a pyrrolidine ring, a tetrahydrofuran ring, a tetrahydrothiophene ring, a piperidine ring, a tetrahydropyran ring, a tetrahydrothiopyran ring, a diketopyrrolopyrrole ring, and the like.

As the aromatic heterocyclic group, an aromatic hetero ring containing at least one selected from a nitrogen atom, an oxygen atom, and a sulfur atom as a ring-constituting hetero atom is preferable. In addition, with regard to the number of ring members of the aromatic hetero ring, a 3- to 8-membered ring is preferable, and a 5- or 6-membered ring is more preferable.

Examples of the 5-membered aromatic heterocyclic group include a pyrrole ring, an imidazole ring, a pyrazole ring, an oxazole ring, a thiazole ring, a triazole ring, a furan ring, a thiophene ring, a selenophene ring, and the like. In addition, examples of the 6-membered aromatic hetero ring include a pyridine ring, a pyrimidine ring, a pyrazine ring, a triazine ring, and the like.

A fused ring group containing at least one aromatic ring means a fused ring group in which at least one aromatic ring (for example, a benzene ring, a naphthalene ring, an anthracene ring, a pentacene ring, an azulene ring, a pyrene ring, and the like) is fused with one or more of other rings selected from, for example, an aliphatic ring and an aliphatic hetero ring.

In addition, the fused ring group containing at least one aromatic hetero ring means a fused ring group in which at least one aromatic hetero ring is fused with one or more of other rings selected from, for example, an aromatic ring, an aliphatic ring, and an aliphatic hetero ring. The aromatic hetero ring is the same as the above-described aromatic heterocyclic group as $D_1$, and the same specific examples are applied.

It is preferable that the organic group represented by $D_1$ further has a substituent. The substituent is not particularly limited, and examples thereof include an alkyl group, a cycloalkyl group, an alkenyl group, an alkynyl group, an aryl group, a heterocyclic group, an alkoxy group, an alkylthio group, an amino group, an alkylamino group, an arylamino group, an acyl group, an alkylcarbonyloxy group, an aryloxy group, an alkoxycarbonyl group, an aryloxycarbonyl group, an acylamino group, a sulfonamide group, a carbamoyl group, a sulfamoyl group, a halogen atom, a cyano group, a hydroxy group, a mercapto group, and a carboxy group.

Specific examples of the fused ring group containing at least one aromatic ring and the fused ring group containing at least one aromatic hetero ring are shown below. These groups may further have a substituent.

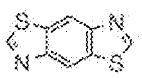 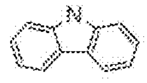 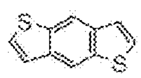 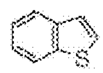

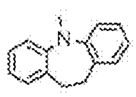 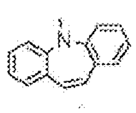 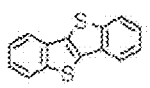 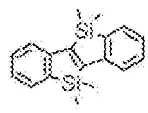

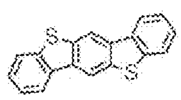 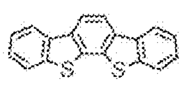 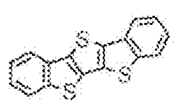

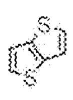 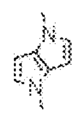 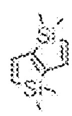 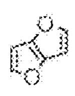 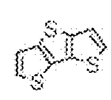 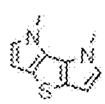

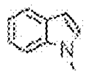 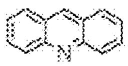 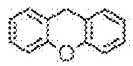 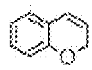 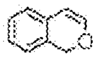

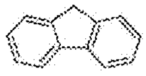

In a case where $D_1$ is an organic group, m1 represents an integer of 1 to 4, preferably represents an integer of 1 to 3, and more preferably represents 2.

In one aspect of the present invention, in the case where $D_1$ is an organic group and m1 is an integer of 2 or more, all substituents represented by Formula 2a are preferably the same as each other.

In a case where $D_1$ is a nitrogen atom, m1 represents an integer of 1 to 3. In the case where $D_1$ is a nitrogen atom and m1 = 1 or 2, the nitrogen atom as $D_1$ means to have two or one hydrogen atom or another substituent different from the substituent represented by Formula 2a. In one aspect of the present invention, in the case where $D_1$ is the nitrogen atom, it is preferable that m1 = 3.

In one aspect of the present invention, in the case where $D_1$ is the nitrogen atom and m1 is an integer of 2 or more, all substituents represented by Formula 2a are preferably the same as each other.

The compound Q is preferably a compound represented by Formula 3 in one aspect.

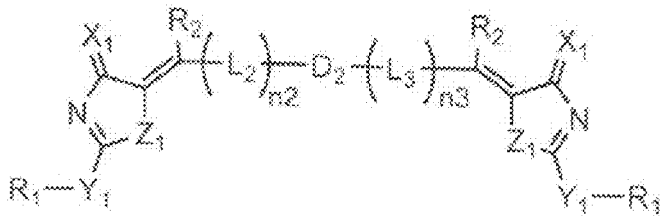

Formula 3

In Formula 3, $X_1$'s each independently represent NR, a sulfur atom, an oxygen atom, a selenium atom, or $CR_3R_4$, $R_1$'s each independently represent a substituent, $R_2$, $R_3$, $R_4$, and R each independently represent a hydrogen atom or a substituent, $L_2$ and $L_3$ each independently represent an alkenylene group, an alkynylene group, an arylene group, or a heteroarylene group, $D_2$ represents a linking group, n2 and n3 each independently represent an integer of 1 to 5, and in a case where n2 is an integer of 2 to 5, a plurality of $L_2$'s may be the same as or different from each other, and in a case where n3 is an integer of 2 to 5, a plurality of $L_3$'s may be the same as or different from each other.

$Z_1$, $Y_1$, $X_1$, $R_1$, and $R_2$ in Formula 3 are the same as $Z_1$, $Y_1$, $X_1$, $R_1$, and $R_2$ in Formula 1 described above.

An alkenylene group, an alkynylene group, an arylene group, and a heteroarylene group as $L_2$ and $L_3$ are the same as the alkenylene group, the alkynylene group, the arylene group, and the heteroarylene group exemplified as the specific examples of the linking group represented by $L_1$ in Formula 2 described above, and the same specific examples are applied. In addition, these groups may further have a substituent, and as the substituent, the specific examples exemplified as the substituent that the linking group represented by $L_1$ in Formula 2 may have been applied.

As described above, n2 and n3 are integers of 1 to 5, are preferably integers of 1 to 4, and more preferably integers of 1 to 3.

Examples of the linking group represented by $D_2$ include an alkenylene group, a cycloalkenylene group, an alkynylene group, an arylene group, a divalent aliphatic heterocyclic group, a heteroarylene group, a divalent fused ring group containing at least one aromatic ring, a divalent fused ring group containing at least one aromatic hetero ring, and the like.

In regard to the alkenylene group, the cycloalkenylene group, the alkynylene group, the arylene group, the divalent heterocyclic group, and the heteroarylene group, the same specific examples as $L_1$ in Formula 2 described above are applied.

The divalent fused ring group containing at least one aromatic ring as $D_2$ is the same as the fused ring group containing at least one aromatic ring as $D_1$ in Formula 2 described above, and the same specific examples are applied.

The divalent fused ring group containing at least one aromatic hetero ring as $D_2$ is the same as the fused ring group containing at least one aromatic hetero ring as $D_1$ in Formula 2 described above, and the same specific examples are applied.

In one aspect of the present invention, the linking group represented by $D_2$ is preferably a divalent fused ring group containing at least one aromatic ring or a divalent fused ring group containing at least one aromatic hetero ring.

In addition, the linking group represented by $D_2$ may further have a substituent, and as the substituent, the specific examples exemplified as the substituent that the organic group represented by $D_1$ in Formula 2 may have been

applied.

In addition, in one aspect of the present invention, substituents represented by Formulas 3a and 3b of Formula 3 are preferably the same as each other.

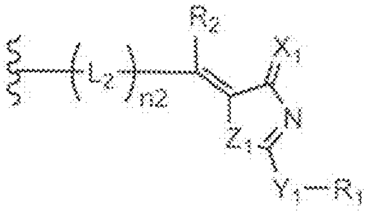

Formula 3a

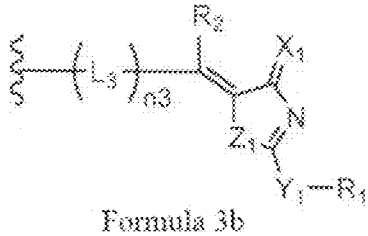

Formula 3b

Specific examples of the compound Q having the at least one structure M represented by Formula 1 are described below, but the compound Q is not limited to these examples. In the following specific examples, "2EH" represents 2-ethylhexyl.

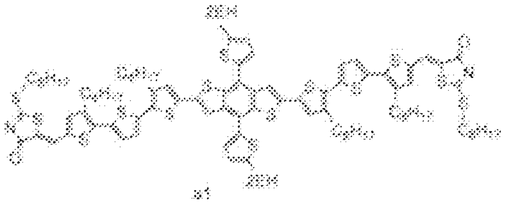

a1

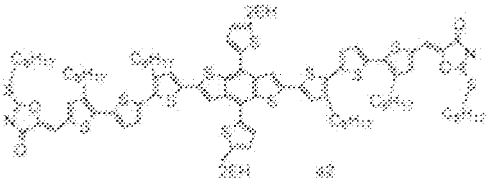

a2

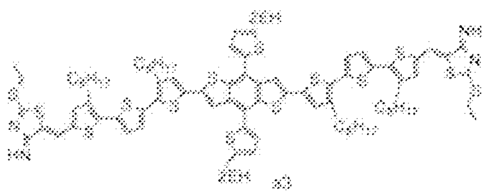

a3

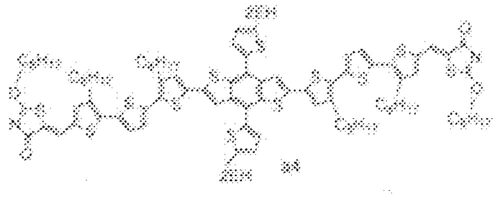

a4

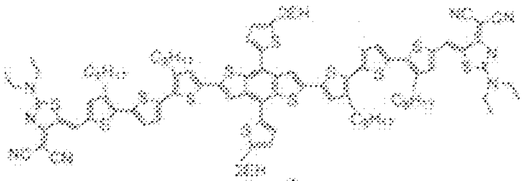

a5

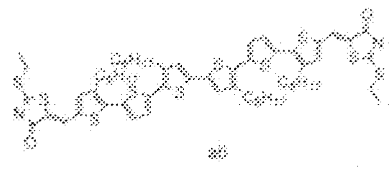

a6

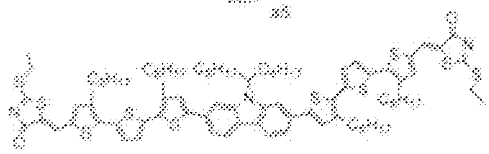

a7

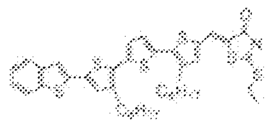

a8

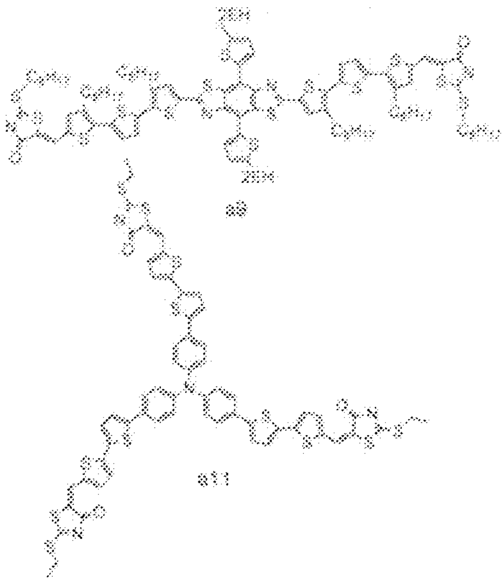

a9

a11

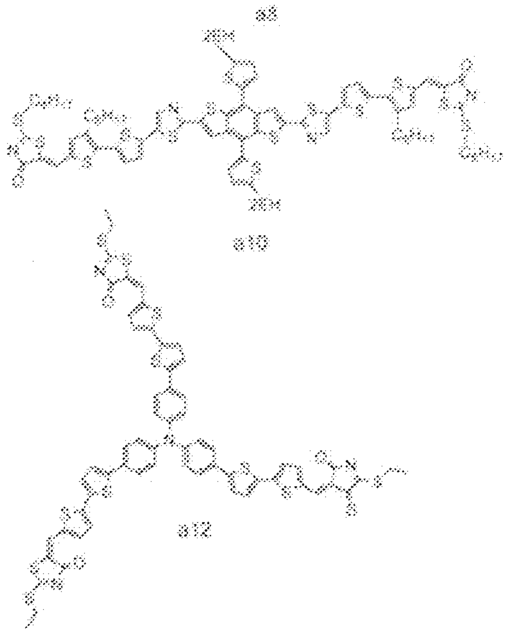

a10

a12

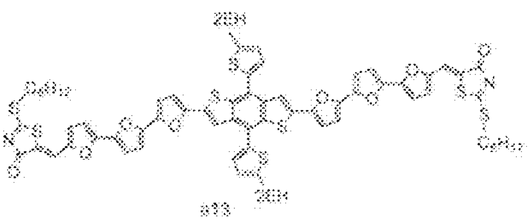

a13

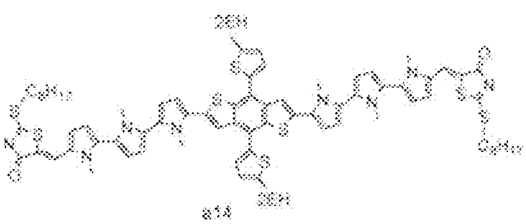

a14

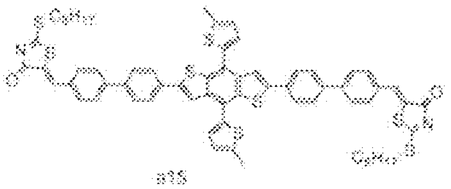
a15

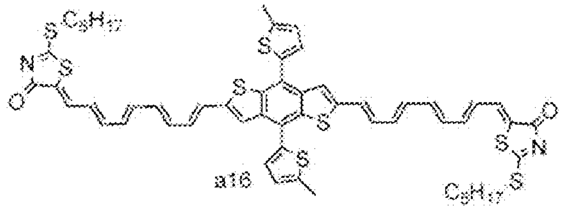
a16

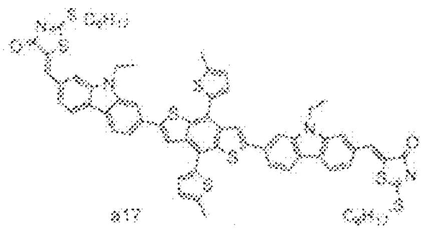
a17

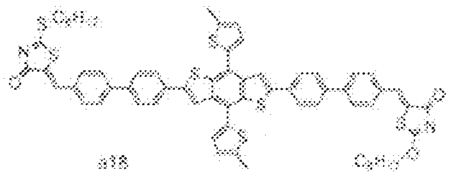
a18

The compound Q having the at least structure M represented by Formula 1 can be synthesized by a known method, and can be synthesized with reference to methods described in the following literatures.

J. Org. Chem., 79, 7766(2014), Journal of Medicinal Chemistry, 50, 1546(2007), European Journal of Organic Chemistry, 425(2007), Org. Lett., 2005, 7, 5253, Macromolecules, 36, 7986(2003), Chemistry of Materials, 22, 1977(2010), Angewandte Chem-International Edition, 45, 3170(2006), Tetorahedron, 68, 767(2012), Org. Lett., 14, 4330(2012), Tetorahedron Letters, 53, 5197(2012), Journal of Materials Chemistry C, 2, 124(2014), J. Org. Chem., 75, 495(2010), European Journal of Organic Chemistry, 1916(2013), Journal of American Chemical Society, 134, 11064(2012), Journal of Materials Chemistry A, 1, 2795(2013), and Journal of Materials Chemistry, 22, 9173(2012).

A hole transporting material that forms the hole transport layer 3 may include a material other than the compound Q. The material may be a liquid material or a solid material without any limitation. Examples of the hole transporting material include inorganic materials such as CuI and CuNCS, organic hole transporting materials described in Paragraphs 0209 to 0212 of JP2001-291534A, and the like. Preferred examples of the organic hole transporting material include conductive polymers such as polythiophene, polyaniline, polypyrrole, and polysilane, spiro compounds in which two rings share a central atom such as C or Si having a tetrahedral structure, aromatic amine compounds such as triarylamine, triphenylene compounds, nitrogen-containing heterocyclic compounds, and liquid-crystalline cyano compounds.

As the hole transporting material that can be included in addition to the compound Q having the at least structure M represented by Formula 1, an organic hole transporting material which can be applied in a solution state and then has a solid shape is preferable, and specific examples thereof include 2,2',7,7'-tetrakis-(N,N-di-p-methoxyphenylamino)-9,9'-spirobifluorene (also referred to as "spiro-OMeTAD"), poly(3-hexylthiophene-2,5-diyl), 4-(diethylamino)benzaldehyde diphenylhydrazone, poly(3,4-ethylene dioxythiophene (PEDOT), and the like.

Although not particularly limited, the film thickness of the hole transport layer 3 is preferably 50 $\mu$m or less, more preferably 1 nm to 10 $\mu$m, still more preferably 5 nm to 5 $\mu$m, and still more preferably 10 nm to 1 $\mu$m. In addition, the film thickness of the hole transport layer 3 corresponds to an average distance between the second electrode 2 and the surface of the photosensitive layer 13, and can be measured by observing a cross-section of the photoelectric conversion element by using a scanning electron microscope (SEM) and the like.

<Second Electrode 2>

The second electrode 2 functions as a positive electrode in a solar cell. The second electrode 2 is not particularly limited as long as the second electrode 2 has conductivity. Typically, the second electrode 2 can be configured to have the same configuration as that of the conductive support 11. In a case where sufficient strength is maintained, the support 11a is not necessary.

As a structure of the second electrode 2, a structure having a high current-collection effect is preferable. At least one of the conductive support 11 or the second electrode 2 needs to be substantially transparent so that light reaches the photosensitive layer 13. In the solar cell of the present invention, it is preferable that the conductive support 11 is transparent and solar light is incident from the support 11a side. In this case, it is more preferable that the second electrode 2 has a light-reflecting property.

Examples of a material used to form the second electrode 2 include metals such as platinum (Pt), gold (Au), nickel (Ni), copper (Cu), silver (Ag), indium (In), ruthenium (Ru), palladium (Pd), rhodium (Rh), iridium (Ir), osmium (Os), and aluminum (Al), the above-described conductive metal oxides, carbon materials, conductive polymers, and the like.

The carbon materials may be conductive materials formed through bonding of carbon atoms, and examples thereof include fullerene, a carbon nanotube, graphite, graphene, and the like.

As the second electrode 2, a thin film (including a thin film obtained through vapor deposition) of a metal or a conductive metal oxide, or a glass substrate or a plastic substrate which has the thin film is preferable. As the glass substrate or the plastic substrate, glass including a gold or platinum thin film or glass on which platinum is vapor-deposited is preferable.

The film thickness of the second electrode 2 is not particularly limited, and is preferably 0.01 to 100 μm, more preferably 0.01 to 10 μm, and still more preferably 0.01 to 1 μm.

<Other Configurations>

In the present invention, a spacer or a separator can also be used instead of the blocking layer 14 and the like or in combination with the blocking layer 14 and the like so as to prevent the first electrode 1 and the second electrode 2 from coming into contact with each other.

In addition, a hole blocking layer may be provided between the second electrode 2 and the hole transport layer 3.

<<Solar Cell>>

The solar cell of the present invention is constituted by using the photoelectric conversion element of the present invention. For example, as illustrated in Fig. 1A, Fig. 2 to Fig. 5, the photoelectric conversion element 10 having a configuration, which is allowed to operate by the external circuit 6, can be used as the solar cell. As the external circuit 6 that is connected to the first electrode 1 (the conductive support 11) and the second electrode 2, a known circuit can be used without particular limitation.

For example, the present invention is applicable to individual solar cells described J. Am. Chem. Soc., 2009, 131(17), p. 6050 to 6051, and Science, 338, p. 643(2012). Furthermore, the present invention can be appropriately applied to dye sensitized solar cells, organic thin film solar cells, and the like.

It is preferable that a lateral surface of the solar cell of the present invention is sealed with a polymer, an adhesive, and the like so as to prevent deterioration, evaporation, and the like in constituent substances.

<< Method of Manufacturing Photoelectric Conversion Element and Solar Cell>>

The photoelectric conversion element and the solar cell of the present invention can be manufactured in accordance with a known method, for example, a method described in J. Am. Chem. Soc., 2009, 131(17), p. 6050 to 6051, Science, 338, p. 643(2012), and the like.

Hereinafter, the method of manufacturing the photoelectric conversion element and the solar cell of the present invention will be described in brief.

In the manufacturing method of the present invention, first, at least one of the blocking layer 14, the porous layer 12, the electron transport layer 15, or the hole transport layer 16 is formed on a surface of the conductive support 11 according to the purpose.

For example, the blocking layer 14 can be formed by a method in which a dispersion, which contains an insulating substance or a precursor compound thereof, and the like, is applied to the surface of the conductive support 11, and the dispersion is baked, a spray pyrolysis method, and the like.

A material that forms the porous layer 12 is preferably used as fine particles, and more preferably a dispersion that contains the fine particles.

A method of forming the porous layer 12 is not particularly limited, and examples thereof include a wet-type method, a dry-type method, and other methods (for example, a method described in Chemical Review, Vol. 110, p. 6595 (published on 2010)). In these methods, it is preferable that the dispersion (paste) is applied to the surface of the conductive support 11 or the surface of the blocking layer 14 and then the dispersion is baked at a temperature 100°C to 800°C for ten minutes to ten hours, for example, in the air. According to this, it is possible to bring the fine particles into close contact with each other.

In a case where baking is performed a plurality of times, a temperature in baking except final baking (a baking temperature except for a final baking temperature) is preferably set to be lower than the temperature in the final firing (the final baking temperature). For example, in a case where titanium oxide paste is used, the baking temperature except for the final baking temperature can be set in a range of 50°C to 300°C. In addition, the final baking temperature can be set in a range of 100°C to 600°C to be higher than the baking temperature except for the final baking temperature. In a case where a glass support is used as the support 11a, the baking temperature is preferably 60°C to 500°C.

The amount of a porous material applied to form the porous layer 12 is appropriately set in correspondence with the film thickness of the porous layer 12, the number of times of coating, and the like, and there is no particular

limitation thereto. For example, the amount of the porous material applied per surface area 1 $m^2$ of the conductive support 11 is preferably 0.5 to 500 g, and more preferably 5 to 100 g.

In a case where the electron transport layer 15 or the hole transport layer 16 is provided, the layer can be formed in the same manner as in the hole transport layer 3 to be described below.

Subsequently, the photosensitive layer 13 is provided.

Examples of a method of providing the photosensitive layer 13 include a wet-type method and a dry-type method, and there is no particular limitation thereto. In the present invention, the wet-type method is preferable, and for example, a method of bringing an arbitrary layer into contact with a light absorbing agent solution that contains a perovskite-type light absorbing agent is preferable. In the method, first, the light absorbing agent solution for forming the photosensitive layer is prepared. The light absorbing agent solution contains $MX_2$ and AX which are raw materials of the perovskite compound. Here, A, M, and X are the same as A, M, and X in Formula (I). In the light absorbing agent solution, a molar ratio between $MX_2$ and AX is appropriately adjusted in correspondence with the purpose. In a case of forming the perovskite compound as the light absorbing agent, the molar ratio between AX and $MX_2$ is preferably 1:1 to 10:1. The light absorbing agent solution can be prepared by mixing $MX_2$ and AX in a predetermined molar ratio and, preferably, by heating the resultant mixture. The formation liquid is typically a solution, but may be a suspension. Heating conditions are not particularly limited. A heating temperature is preferably 30°C to 200°C, and more preferably 70°C to 150°C. A heating time is preferably 0.5 to 100 hours, and more preferably 1 to 3 hours. As a solvent or a dispersion medium, the following solvent or dispersion medium can be used.

Then, the light absorbing agent solution, which is prepared, is brought into contact with a surface of a layer (in the photoelectric conversion element 10, a layer of any one of the porous layer 12, the blocking layer 14, the electron transport layer 15, and the hole transport layer 16) on which the photosensitive layer 13 is to be formed. Specifically, application of the light absorbing agent solution or immersion in the light absorbing agent solution is preferable. A contact temperature is preferably 5°C to 100°C, and immersion time is preferably 5 seconds to 24 hours and more preferably 20 seconds to 1 hour. In a case of drying the light absorbing agent solution that is applied, with regard to the drying, drying with heat is preferable, and drying is performed by heating the applied light absorbing agent solution typically at 20°C to 300°C, and preferably at 50°C to 170°C.

In addition, the photosensitive layer can also be formed in conformity to a method of synthesizing the perovskite compound.

In addition, another example of the method includes a method in which an AX solution that contains AX, and an $MX_2$ solution that contains $MX_2$ are each independently applied (including an immersion method), and are dried as necessary. In this method, an arbitrary solution may be applied in advance, but it is preferable that the $MX_2$ solution is applied in advance. A molar ratio between AX and $MX_2$, application conditions, and drying conditions in this method are the same as in the above-described method. In this method, AX or $MX_2$ may be vapor-deposited instead of application of the AX solution and the $MX_2$ solution.

Still another example of the method includes a dry-type method such as a vacuum deposition by using a compound or a mixture from which a solvent of the light absorbing agent solution is removed. For example, a method of simultaneously or sequentially vapor-depositing AX and $MX_2$ may be exemplified.

According to the methods and the like, the perovskite compound is formed on the surface of the porous layer 12, the blocking layer 14, the electron transport layer 15, or the hole transport layer 16 as the photosensitive layer.

The hole transport layer 3 is formed on the photosensitive layer 13 that is provided as described above.

The hole transport layer 3 can be formed through application and drying of a hole transporting material solution that contains a hole transporting material. In the hole transporting material solution, a concentration of the hole transporting material is preferably 0.1 to 1.0 M (mol/L) when considering that application properties are excellent, and in a case of providing the porous layer 12, the hole transporting material solution easily intrudes into pores of the porous layer 12.

After the hole transport layer 3 is formed, the second electrode 2 is formed, thereby manufacturing the photoelectric conversion element.

The film thicknesses of the respective layers can be adjusted by appropriately changing the concentrations of respective dispersion liquids or solutions and the number of times of application. For example, in a case where the photosensitive layers 13B and 13C having a large film thickness are provided, a light absorbing agent solution may be applied and dried a plurality of times.

The respective dispersion liquids and solutions described above may respectively contain additives such as a dispersion auxiliary agent and a surfactant as necessary.

Examples of the solvent or dispersion medium that is used in the method of manufacturing the photoelectric conversion element include a solvent described in JP2001-291534A, but the solvent or dispersion medium is not particularly limited thereto. In the present invention, an organic solvent is preferable, and an alcohol solvent, an amide solvent, a nitrile solvent, a hydrocarbon solvent, a lactone solvent, a halogen solvent, and a mixed solvent of two or more kinds thereof are preferable. As the mixed solvent, a mixed solvent of the alcohol solvent and a solvent selected from the amide solvent, the nitrile solvent, and the hydrocarbon solvent is preferable. Specifically, methanol, ethanol,

isopropanol, γ-butyrolactone, chlorobenzene, acetonitrile, N,N'-dimethylformamide (DMF), dimethylacetamide, and a mixed solvent thereof are preferable.

A method of applying the solutions or dispersants which form the respective layers is not particularly limited, and it is possible to use a known application method such as spin coating, extrusion die coating, blade coating, bar coating, screen printing, stencil printing, roll coating, curtain coating, spray coating, dip coating, an inkjet printing method, and an immersion method. Among these, spin coating, screen printing, and the like are preferable.

The photoelectric conversion element of the present invention may be subjected to an efficiency stabilizing treatment such as annealing, light soaking, and being left as is in an oxygen atmosphere as necessary.

The photoelectric conversion element prepared as described above can be used as a solar cell after connecting the external circuit 6 to the first electrode 1 and the second electrode 2.

Examples

Hereinafter, examples of the present invention will be described in detail, but the content of the present invention is not limited thereto.

<Synthesis of Hole Transporting Material>

[Synthesis of Compound a1]

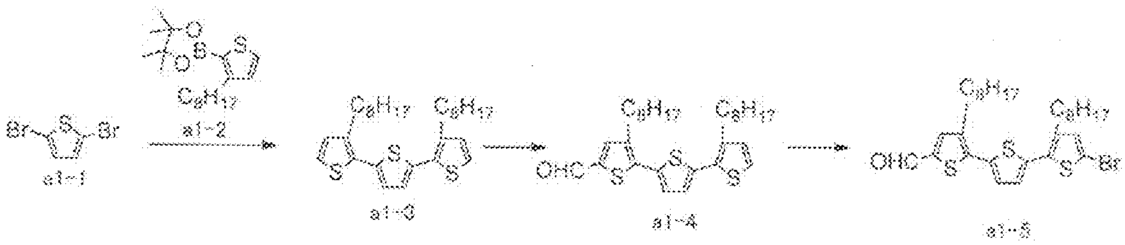

a1-1 a1-3 a1-4 a1-5

• Synthesis of Compound a1-3

12 g of dibromothiophene (a1-1) (product of Tokyo Chemical Industry Co., Ltd.) and 32 g of compound a1-2 were mixed in 50 mL of toluene/methanol (volume ratio: 1/1). Subsequently, 274 mg of PEPSI™ (Pyridine-Enhanced Precatalyst Preparation Stabilization and Initiation)-iPr (product of Sigma-Aldrich Co. LLC.) and 35 g of $K_2CO_3$ were added to the resultant mixture. The mixture after the addition was heated to 70°C and was stirred for 8 hours in a nitrogen atmosphere. The resultant stirred mixture was left to cool down to room temperature, and water and ethyl acetate were added to the resultant cooled down mixture. The mixture after addition was subject to liquid separation to concentrate an organic layer. The organic layer was purified with column chromatography, thereby obtaining 10 g of compound a1-3.

A structure of the compound a1-3 was confirmed with mass spectrometry (MS).
MS-ESI m/z = 473.2 $(M+H)^+$

• Synthesis of Compound a1-4

10 mL of phosphorus oxychloride was added dropwise to 20 mL of DMF that was cooled down to 0°C, and the resultant mixture was stirred for 30 minutes. Thereafter, 4.5 g of compound a1-3 was added to the stirred mixture. The mixture after addition was heated to 45°C and then was stirred for 5 hours. This reaction solution was added dropwise to water maintained at 30°C, and the resultant mixture was stirred for 1 hour. Ethyl acetate was added to the resultant stirred mixture. The mixture after addition was subjected to liquid separation to concentrate an organic layer. Continuously, the concentrate was purified with column chromatography, thereby obtaining 4 g of compound a1-4.

A structure of the compound a1-4 was confirmed with mass spectrometry (MS).
MS-ESI m/z=501.2 $(M+H)^+$

• Synthesis of Compound a1-5

N-bromosuccinimide (NBS) (530 mg) was added to a DMF (20 mL) solution of the compound a1-4 (1.5 g) cooled down to 0°C, and the resultant mixture was left to cool down to room temperature, and was stirred for 3 hours.

Water and ethyl acetate were added to the resultant stirred mixture. The mixture after addition was subjected to liquid separation to concentrate an organic layer, and then the organic layer was purified with column chromatography, thereby obtaining 1 g of compound a1-5.

A structure of the compound a1-5 was confirmed with mass spectrometry (MS).
MS-ESI m/z = 581.1 (M+H)$^+$

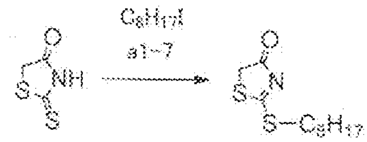


a1-6 a1-8

• Synthesis of Compound a1-8

6.7 g of compound a1-6 and 13.8 g of potassium carbonate were mixed in 80 mL of dimethylacetamide, compound a1-7 (12 g) was added to the resultant mixture, and then the mixture after addition was stirred for 5 hours. Water and ethyl acetate were added to the resultant stirred mixture to concentrate an organic layer. The resultant concentrate was purified with column chromatography, thereby obtaining 3.8 g of compound a1-8.

A structure of the compound a1-8 was confirmed with mass spectrometry (MS).
MS-ESI m/z = 245.2 (M+H)$^+$

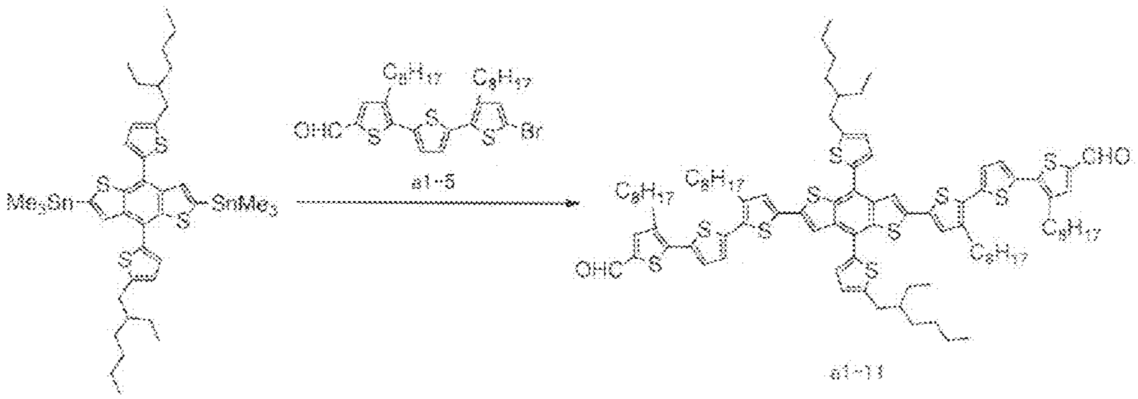

a1-5

a1-10 a1-11

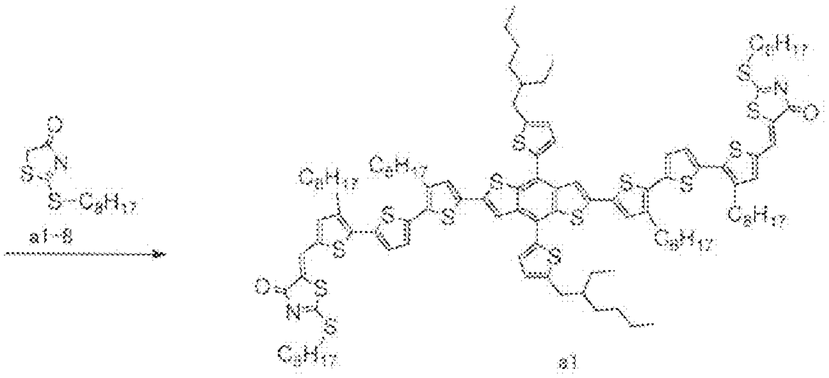

a1-8

a1

• Synthesis of Compound a1-11

Compound a1-10 (800 mg) and 595 mg of compound a1-5 were mixed in toluene (50 mL). Subsequently, $Pd(PPh_3)_4$ (30 mg) was added to the resultant mixture. The mixture after addition was heated to 100°C and stirred for 10 hours. The resultant stirred mixture was left to cool down to room temperature, and water and chloroform were added to the resultant cooled down mixture. The mixture after addition was subjected to liquid separation to concentrate an organic layer, and the organic layer was purified with column chromatography, thereby obtaining 0.6 g of compound a1-11.

A structure of the compound a1-11 was confirmed with mass spectrometry (MS).

MS-ESI m/z = 1576.6 $(M+H)^+$

• Synthesis of Compound a1

0.6 g of compound a1-11 and 0.6 g of compound a1-8 were mixed in 50 mL of chloroform. Subsequently, 5 drops of triethylamine was added to the resultant mixture. The mixture after addition was heated for heating under reflux. In this state, the mixture was stirred for one night. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.15 g of compound a1.

A structure of the compound a1 was confirmed with mass spectrometry (MS).
MS-ESI m/z = 2030.8 $(M+H)^+$

[Synthesis of Compound a2]

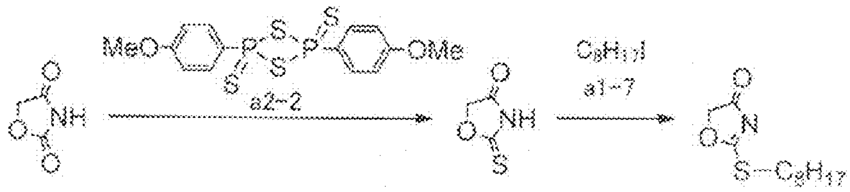


a2-1 a2-3 a2-4

• Synthesis of Compound a2-3

0.28 g of compound a2-1 and 0.7 g of compound a2-2 were mixed in 50 mL of toluene. The resultant mixture was subjected to heating under reflux. In this state, the mixture was stirred for 10 hours. The resultant stirred mixture was left to cool down to room temperature, and water was added to the resultant cooled down mixture. Extraction was performed with dichloromethane, and the resultant extracted material was concentrated. Continuously, the concentrate was purified with column chromatography, thereby obtaining 0.12 g of compound a2-3.

A structure of the compound a2-3 was confirmed with mass spectrometry (MS).
MS-ESI m/z = 118.0 $(M+H)^+$

• Synthesis of Compound a2-4

0.12 g of compound a2-3 and 1.3 g of potassium carbonate were mixed in 10 mL of dimethylacetamide. Subsequently, compound a1-7 (1.2 g) was added to the resultant mixture, and then the mixture after addition was stirred for 5 hours. Water and ethyl acetate were added to the obtained solution to concentrate an organic layer. The resultant concentrate was purified with column chromatography, thereby obtaining 0.10 g of compound a2-4.

A structure of the compound a2-4 was confirmed with mass spectrometry (MS).
MS-ESI m/z = 230.1 $(M+H)^+$

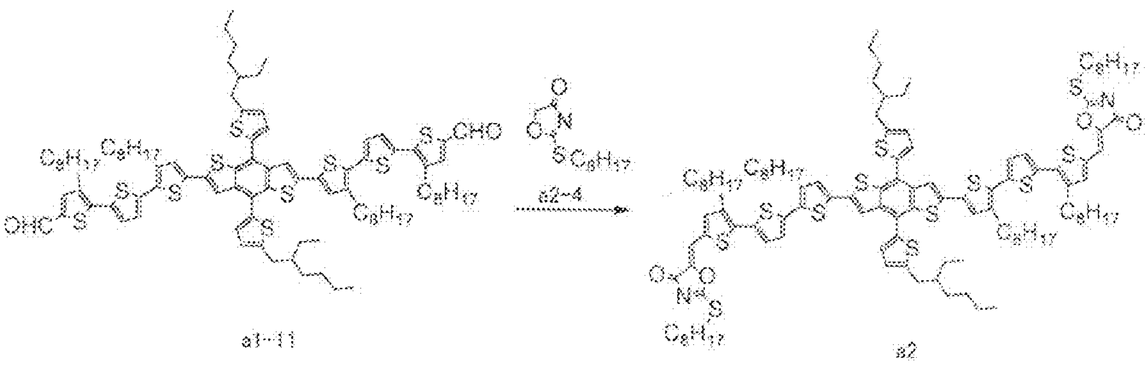


a1-11 a2

• Synthesis of Compound a2

0.1 g of compound a1-11 and 0.1 g of compound a2-4 were mixed in 20 mL of chloroform. Subsequently, 5 drops of triethylamine was added to the resultant mixture. The mixture after addition was heated for heating under reflux. In this state, the mixture was stirred for two nights. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.04 g of compound a2.

A structure of the compound a2 was confirmed with mass spectrometry (MS).
MS-ESI m/z = 1998.8 $(M+H)^+$

[Synthesis of Compound a4]

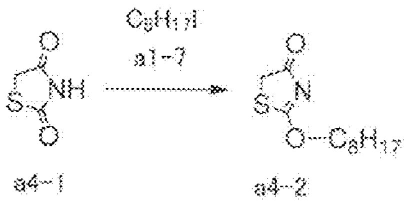

a4-1 a4-2

• Synthesis of Compound a4-2

5 g of compound a4-1 and 10 g of potassium carbonate were mixed in 70 mL of dimethylacetamide, compound a1-7 (10 g) was added to the resultant mixture, and then the mixture after addition was stirred for 7 hours. Water and ethyl acetate were added to the obtained solution to concentrate an organic layer. The resultant concentrate was purified with column chromatography, thereby obtaining 2.1 g of compound a4-2.

A structure of the compound a4-2 was confirmed with mass spectrometry (MS).
MS-ESI m/z = 230.1 $(M+H)^+$

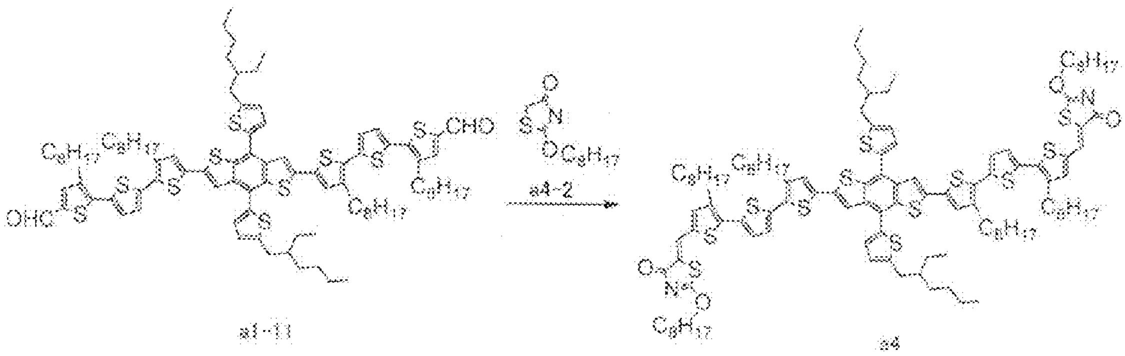

a1-11 a4

• Synthesis of Compound a4

0.6 g of compound a1-11 and 1.2 g of compound a4-2 were mixed in 30 mL of chloroform. Subsequently, 5 drops of triethylamine was added to the resultant mixture. The mixture after addition was heated for heating under reflux. In this state, the mixture was stirred for one night. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.2g of compound a4.

A structure of the compound a4 was confirmed with mass spectrometry (MS).
MS-ESI m/z = 1998.8 $(M+H)^+$

[Synthesis of Compound a5]

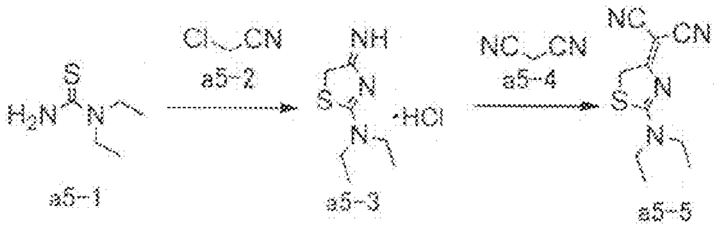


• Synthesis of Compound a5-3

5.2 g of compound a5-1 and 7.2 g of compound a5-2 were mixed in 70 mL of ethanol. The resultant mixture was subjected to heating under reflux. In this state, the mixture was stirred for 3 hours. Thereafter, the resultant stirred mixture was cooled down to 0°C, and a precipitated crystal was filtered, thereby obtaining 2.1 g of compound a5-3.
A structure of the compound a5-3 was confirmed with mass spectrometry (MS).
MS-ESI m/z = 172.1 $(M+H)^+$

• Synthesis of Compound a5-5

2.1 g of compound a5-3 and 0.9 g of compound a5-4 were mixed in 30 mL of 2N NaOH aqueous solution. The resultant mixture was stirred for 3 hours. Thereafter, the resultant stirred mixture was cooled down to 0°C, and a precipitated crystal was filtered, thereby obtaining 0.9 g of compound a5-5.
A structure of the compound a5-5 was confirmed with mass spectrometry (MS).
MS-ESI m/z = 221.1 $(M+H)^+$

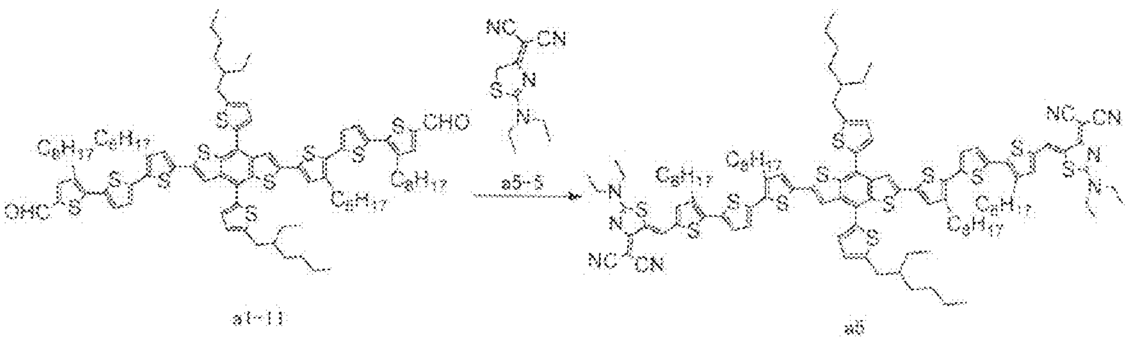


• Synthesis of Compound a5

0.3 g of compound a1-11 and 0.9 g of compound a5-5 were mixed in 20 mL of chloroform. Subsequently, 5 drops of triethylamine was added to the resultant mixture. The mixture after addition was heated for heating under reflux. In this state, the mixture was stirred for two nights. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.04 g of compound a5.
A structure of the compound a5 was confirmed with mass spectrometry (MS).
MS-ESI m/z = 1980.8 $(M+H)^+$

[Synthesis of Compound a6]

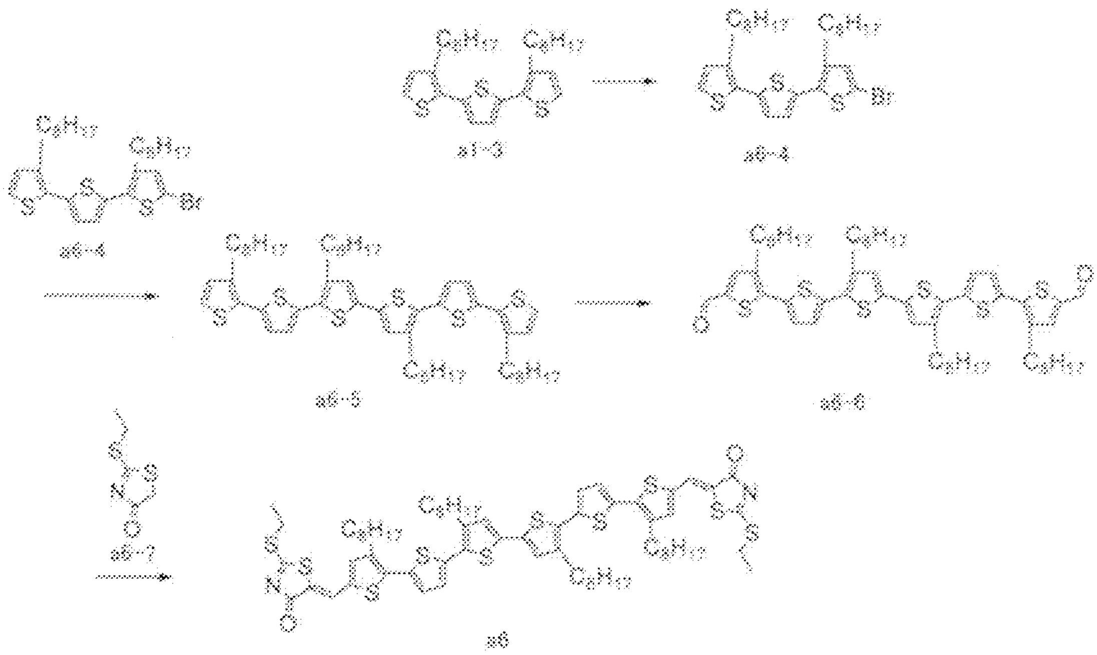

a1-3
a6-4
a6-4
a6-5
a6-6
a6-7
a6

• Synthesis of Compound a6-4

NBS (1.8 g) was added to a DMF (50 mL) solution of compound a1-3 (4.7 g) cooled down to 0°C, and the resultant mixture was left to cool down to room temperature, and was stirred for 3 hours. Water and ethyl acetate were added to the obtained solution. The solution after addition was subjected to liquid separation to concentrate an organic layer, and then the organic layer was purified with column chromatography, thereby obtaining 1.5 g of compound a6-4.

A structure of the compound a6-4 was confirmed with mass spectrometry (MS).
MS-ESI m/z = 553.1 $(M+H)^+$

• Synthesis of Compound a6-5

A tetrahydrofuran (30 mL) solution of compound a6-4 (1.1 g) was cooled down to -78°C, and 1.3 mL of nBuLi (1.6 M hexane solution) was added dropwise to the resultant solution. The solution after addition was stirred for 1 hour. Continuously, $Me_3SnCl$ (0.4 g) was added to the resultant stirred solution, and the resultant mixture was left to cool down to room temperature. The resultant cooled down mixture was stirred for 4 hours. Water and ethyl acetate were added to the resultant stirred mixture to concentrate an organic layer. The resultant concentrate and 1.2 g of compound a6-4 were mixed in toluene (80 mL), and $Pd(PPh_3)_4$ (200 mg) was added to the resultant mixture. The mixture after addition was heated to 100°C, and was stirred for 10 hours. The resultant stirred mixture was left to cool down to room temperature, and water and chloroform were added to the resultant cooled down mixture. The mixture after addition was subjected to liquid separation to concentrate an organic layer, and the organic layer was purified with column chromatography, thereby obtaining 1.0 g of compound a6-5.

A structure of the compound a6-5 was confirmed with mass spectrometry (MS).
MS-ESI m/z = 923.4 $(M+H)^+$

• Synthesis of Compound a6-6

5 mL of phosphorus oxychloride was added dropwise to 15 mL of DMF that was cooled down to 0°C, and the resultant mixture was stirred for 30 minutes. 1.0 g of compound a6-5 was added to the resultant stirred mixture. The mixture after addition was heated to 45°C and was stirred for 8 hours. This reaction solution was added dropwise to water maintained at 30°C, and the resultant mixture was stirred for 1 hour. Ethyl acetate was added to the resultant stirred mixture. The mixture after addition was subjected to liquid separation to concentrate an organic layer. Continuously, the concentrate was purified with column chromatography, thereby obtaining 0.5 g of compound a6-6.

A structure of the compound a6-6 was confirmed with mass spectrometry (MS).
MS-ESI m/z = 999.4 $(M+H)^+$

• Synthesis of Compound a6

0.5 g of compound a6-6 and 0.5 g of compound a6-7 were mixed in 10 mL of chloroform. Subsequently, 5 drops of triethylamine was added to the resultant mixture. The mixture after addition was heated for heating under reflux. In this state, the mixture was stirred for two nights. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.1 g of compound a6.

A structure of the compound a6 was confirmed with mass spectrometry (MS).
MS-ESI m/z = 1285.4 $(M+H)^+$

[Synthesis of Compound a7]

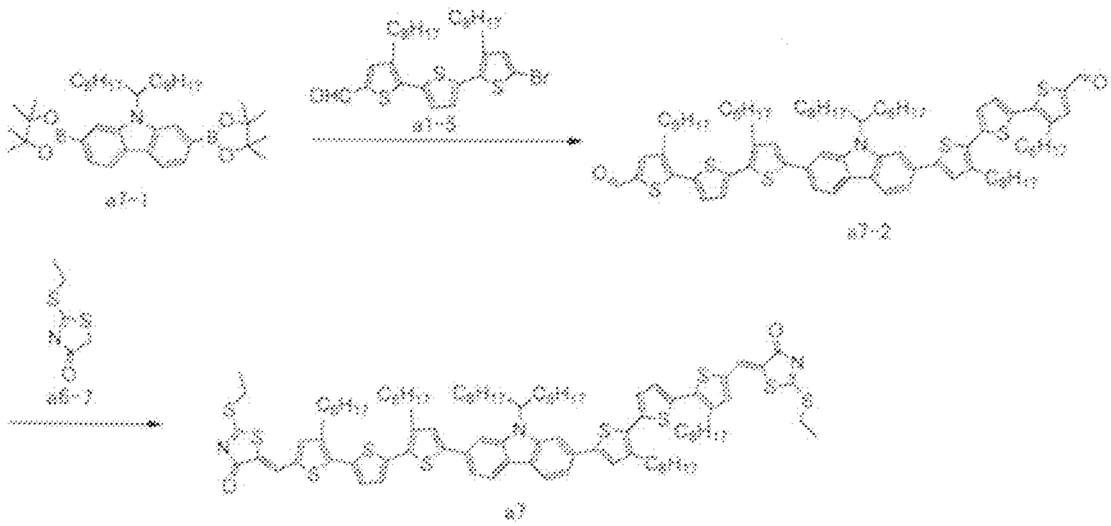

a7-1

a1-5

a7-2

a6-7

a7

• Synthesis of Compound a7-2

3.3 g of compound a7-1 and 11.2 g of compound a1-5 were mixed in 70 mL of toluene. Subsequently, 200 mg of $Pd(PPh_3)_4$ and 6.9 g of $K_2CO_3$ were added to the resultant mixture. The mixture after addition was heated to 100°C and stirred for 12 hours in a nitrogen atmosphere. The resultant stirred mixture was left to cool down to room temperature, and water and ethyl acetate were added to the resultant cooled down mixture. The mixture after addition was subject to liquid separation to concentrate an organic layer. The organic layer was purified with column chromatography, thereby obtaining 2.7 g of compound a7-2.

A structure of the compound a7-2 was confirmed with mass spectrometry (MS).
MS-ESI m/z = 1403.8 $(M+H)^+$

• Synthesis of Compound a7

2 g of compound a7-2 and 2 g of compound a6-7 were mixed in 20 mL of chloroform. Subsequently, 5 drops of triethylamine was added to the resultant mixture. The mixture after addition was subjected to heating under reflux. In this state, the mixture was stirred for two nights. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.5 g of compound a7.

A structure of the compound a7 was confirmed with mass spectrometry (MS).
MS-ESI m/z = 1689.7 $(M+H)^+$

[Synthesis of Compound a9]

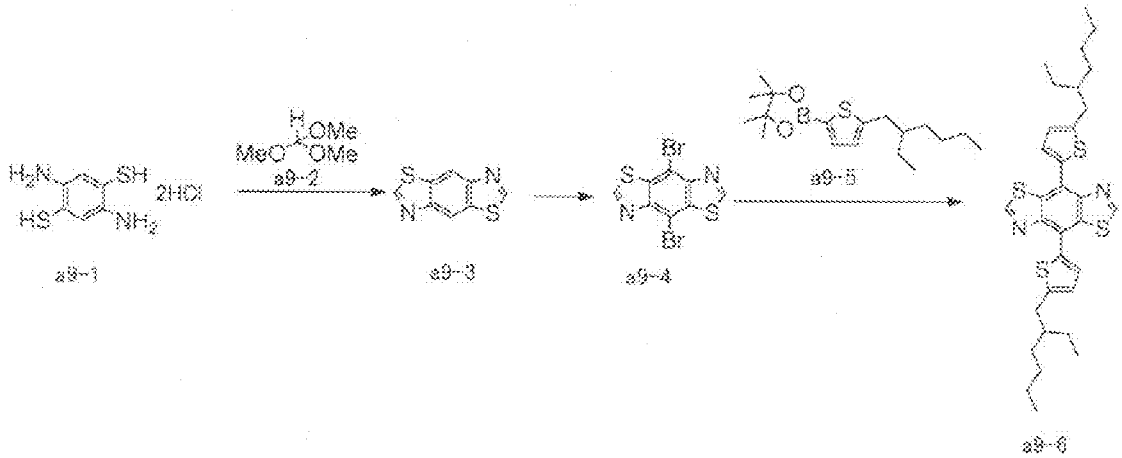


a9-1 a9-3 a9-4 a9-6

• Synthesis of Compound a9-4

15 g of compound a9-1 and 80 mL of compound a9-2 were mixed with each other, and sulfuric acid (1 mL) was added to the resultant mixture. The mixture was heated to 75°C and was stirred for 8 hours. The resultant stirred mixture was left to cool down to room temperature, and water was added to the resultant cooled down mixture. The mixture after addition was filtered, thereby obtaining 10 g of coarse of compound a9-3.

7 g of the coarse of compound a9-3 and 30 g of bromine were mixed in 160 mL of DMF, and the resultant mixture was heated to 75°C and was stirred for 2 hours. The resultant stirred mixture was left to cool down to room temperature, and this reaction solution was added dropwise to a saturated $NaHSO_3$ solution, and the resultant material was stirred for 1 hour and was filtered, thereby obtaining 11 g of compound a9-4.

A structure of the compound a9-4 was confirmed with mass spectrometry (MS).
MS-ESI m/z = 349.8 $(M+H)^+$

• Synthesis of Compound a9-6

1.8 g of compound a9-4 and 4.1 g of compound a9-5 were mixed in 50 mL of tetrahydrofuran. Subsequently, 2-dicyclohexylphosphino-2',6'-dimethoxybiphenyl (1 g), palladium acetate (120 mg), and tripotassium phosphate (4.5 g) were added to the resultant mixture. The mixture after addition was heated to 70°C, and was stirred for 6 hours. The resultant stirred mixture was left to cool down to room temperature, and water and ethyl acetate were added to the resultant cooled down mixture, and the mixture after addition was filtered, thereby obtaining 2 g of compound a9-6.

A structure of the compound a9-6 was confirmed with mass spectrometry (MS).
MS-ESI m/z = 581.2 $(M+H)^+$

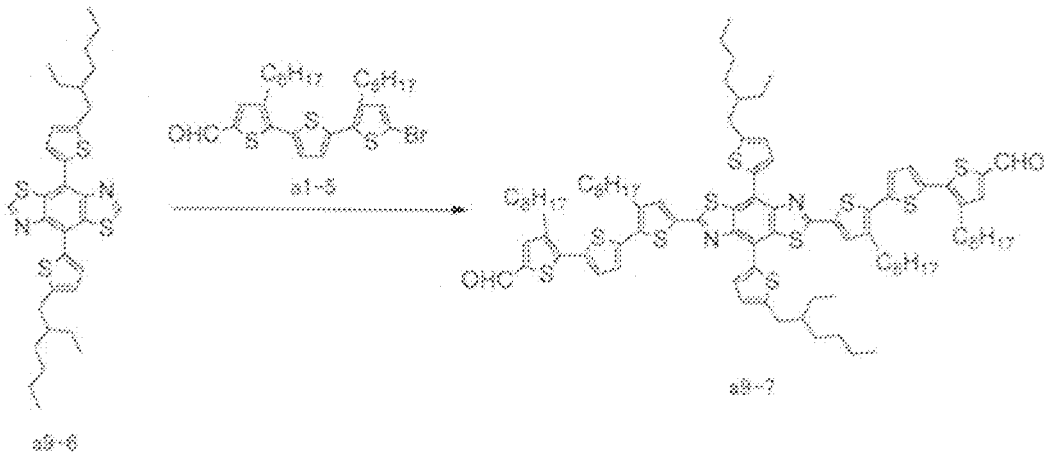


a9-6

a9-7

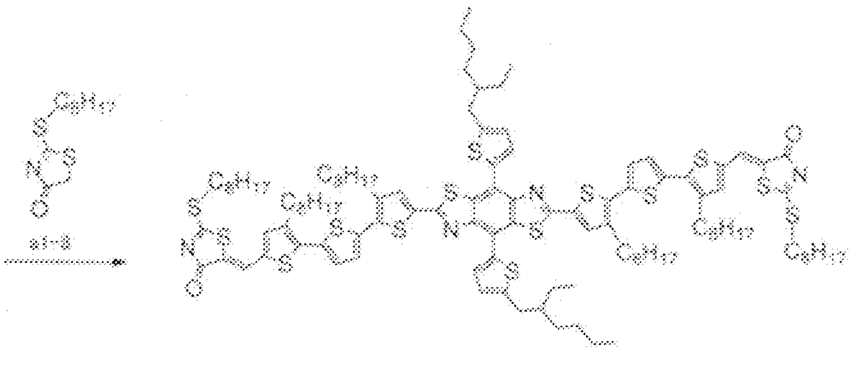


a9

• Synthesis of Compound a9-7

A tetrahydrofuran (20 mL) solution of compound a9-6 (870 mg) was cooled down to -78°C, and 2 mL of nBuLi (1.6 M hexane solution) was added dropwise to the resultant solution. The solution after addition was stirred for 1 hour. Continuously, $Me_3SnCl$ (0.6 g) was added to the resultant stirred solution, and the resultant mixture was left to cool down to room temperature. The resultant cooled down mixture was stirred for 4 hours. Water and ethyl acetate were added to the resultant stirred mixture to concentrate an organic layer. The resultant concentrate and 1.7 g of compound a1-5 were mixed in toluene (80 mL). Subsequently, $Pd(PPh_3)_4$ (220 mg) was added to the resultant mixture. The mixture after addition was heated to 100°C and stirred for 10 hours. The resultant stirred mixture was left to cool down to room temperature, and water and chloroform were added to the resultant cooled down mixture. The mixture after addition was subjected to liquid separation to concentrate an organic layer, and the organic layer was purified with column chromatography, thereby obtaining 0.6 g of compound a9-7.

A structure of the compound a9-7 was confirmed with mass spectrometry (MS).

MS-ESI m/z = 1577.6 $(M+H)^+$

• Synthesis of Compound a9

0.5 g of compound a9-7 and 0.5 g of compound a1-8 were mixed in 20 mL of chloroform. Subsequently, 5 drops of triethylamine was added to the resultant mixture. The mixture after addition was heated for heating under reflux. In this state, the mixture was stirred for one night. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.2 g of compound a9.

A structure of the compound a9 was confirmed with mass spectrometry (MS).

MS-ESI m/z = 2042.8 $(M+H)^+$

[Synthesis of Compound a10]

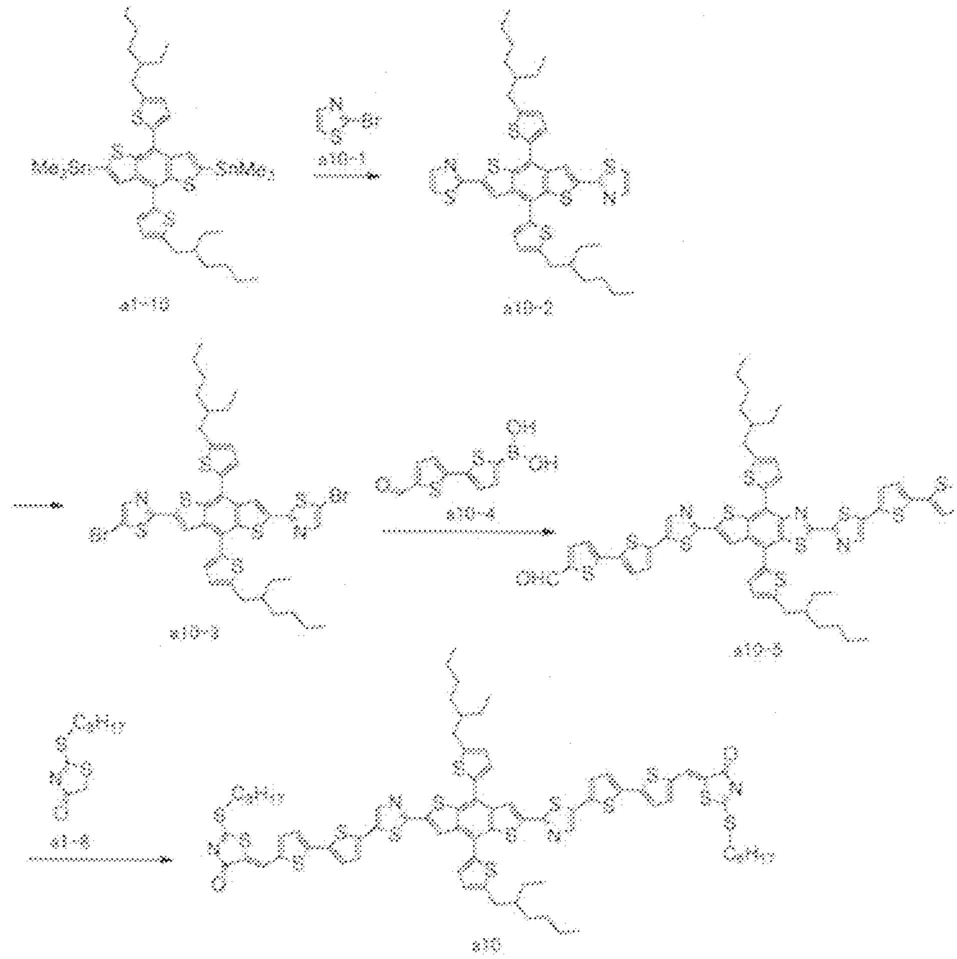


• Synthesis of Compound a10-2

Compound a1-10 (1.8 g) and 7 g of compound a10-1 were mixed in toluene (50 mL). Subsequently, $Pd(PPh_3)_4$ (50 mg) was added to the resultant mixture. The mixture after addition was heated to 100°C and stirred for 10 hours. The resultant stirred mixture was left to cool down to room temperature, and water and chloroform were added to the resultant cooled down mixture. The mixture after addition was subjected to liquid separation to concentrate an organic layer. Continuously, the concentrate was purified with column chromatography, thereby obtaining 1.3 g of compound a10-2.

A structure of the compound a10-2 was confirmed with mass spectrometry (MS).
MS-ESI m/z = 745.2 $(M+H)^+$

• Synthesis of Compound a10-3

NBS (0.7 g) was added to a DMF (20 mL) solution of compound a10-2 (1.3 g) cooled down to 0°C, and the resultant mixture was left to cool down to room temperature, and was stirred for 4 hours. Water and ethyl acetate were added to the obtained solution. The solution after addition was subjected to liquid separation to concentrate an organic layer, and then the organic layer was purified with column chromatography, thereby obtaining 1.1 g of compound a10-3.

A structure of the compound a10-3 was confirmed with mass spectrometry (MS).
MS-ESI m/z = 903.0 $(M+H)^+$

• Synthesis of Compound a10-5

1.1 g of compound a10-3 and 0.9 g of compound a10-4 were mixed in DMF (50 mL). Subsequently, $Pd(PPh_3)_4$ (200 mg) was added to the resultant mixture. The mixture after addition was heated to 100°C and stirred for 10 hours. The resultant stirred mixture was left to cool down to room temperature, and water and chloroform were added to the resultant cooled down mixture. The mixture after addition was subjected to liquid separation to concentrate an organic

layer. Continuously, the concentrate was purified with column chromatography, thereby obtaining 0.5 g of compound a10-5.

A structure of the compound a10-5 was confirmed with mass spectrometry (MS).
MS-ESI m/z = 1130.1 $(M+H)^+$

• Synthesis of Compound a10

0.4 g of compound a10-5 and 0.5 g of compound a1-8 were mixed in 20 mL of chloroform. Subsequently, a minute amount of triethylamine was added to the resultant mixture. The mixture after addition was subjected to heating under reflux. In this state, the mixture was stirred for three nights. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.2 g of compound a10.

A structure of the compound a10 was confirmed with mass spectrometry (MS).
MS-ESI m/z = 1583.3 $(M+H)^+$

Another compound (hole transporting material) was synthesized by combining a method conforming to the above description and a known method.

<Hole Transporting Material>

As the hole transporting material of the present invention, the following compounds were used.

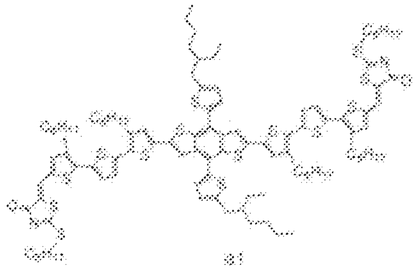
a1

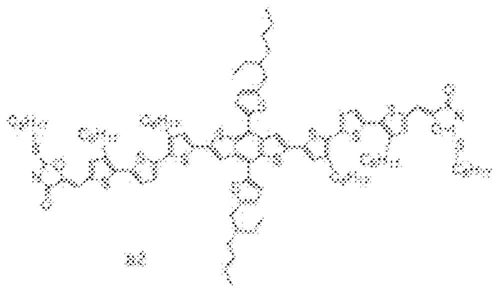
a2

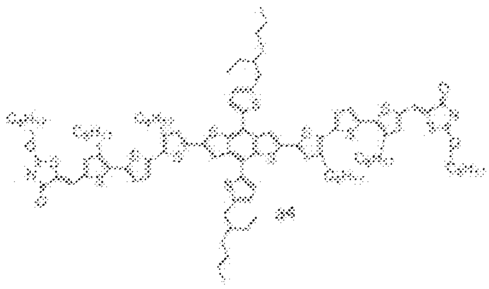
a4

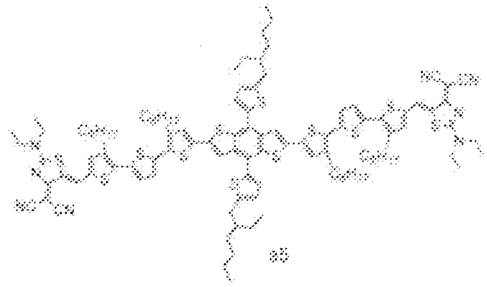
a5

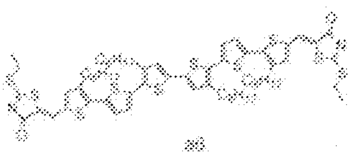
a6

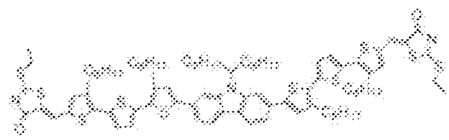
a7

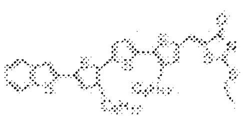
a8

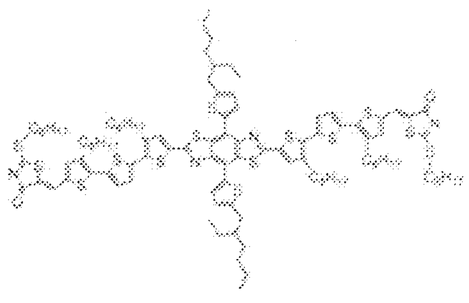
a9

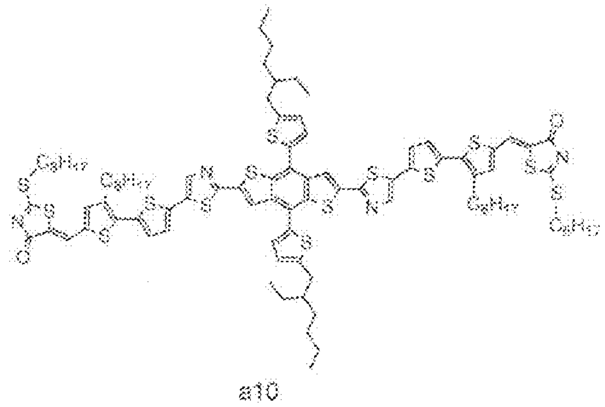

a10

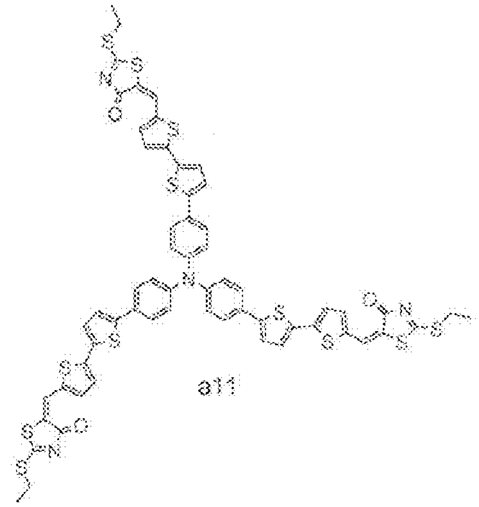

a11

[Hole Transporting Material for Comparison]

As a hole transporting material for comparison, the following compounds b1 and b2, and spiro-MeOTAD (manufactured by 1-material inc.) were used.

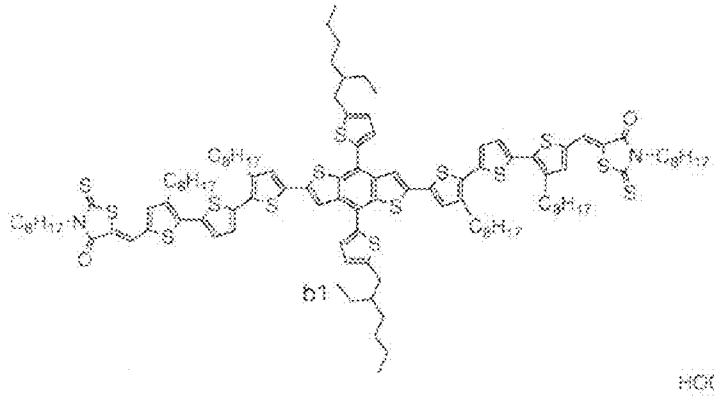

b1

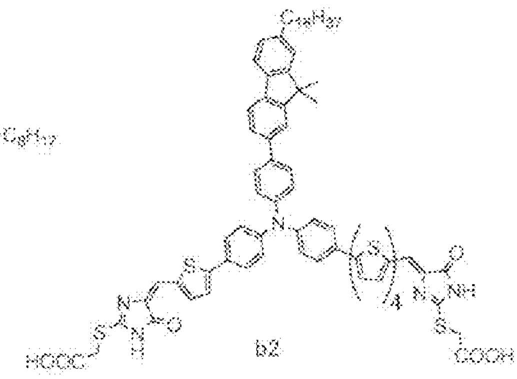

b2

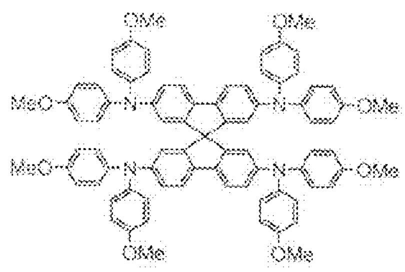

spiro-MeOTAD

The photoelectric conversion element 10A illustrated in Fig. 1A was manufactured in the following procedure. In a case where the film thickness of the photosensitive layer 13 is large, this case corresponds to the photoelectric conversion element 10B illustrated in Fig. 2.

[Manufacturing of Photoelectric Conversion Element (Sample No. 101)]

<Preparation of Conductive Support 11>

A fluorine-doped $SnO_2$ conductive film (transparent electrode 11b, film thickness: 300 nm) was formed on a glass substrate (support 11a, thickness: 2 mm) to prepare the conductive support 11.

<Preparation of Solution for Blocking Layer>

15% by mass isopropanol solution (manufactured by Sigma-Aldrich Co. LLC) of titanium diisopropoxide bis(acetylacetonate) was diluted with 1-butanol, thereby preparing 0.02 M solution for a blocking layer.

<Formation of Blocking Layer 14>

The blocking layer 14 (film thickness: 50 nm) formed from titanium oxide was formed on the $SnO_2$ conductive film of the conductive support 11 by using the prepared 0.02 M solution for the blocking layer at 450°C in accordance with a spray pyrolysis method.

<Preparation of Titanium Oxide Paste>

Ethyl cellulose, lauric acid, and terpineol were added to an ethanol dispersion liquid of titanium oxide (anatase, average particle size: 20 nm), thereby preparing titanium oxide paste.

<Formation of Porous Layer 12>

The prepared titanium oxide paste was applied onto the blocking layer 14 with a screen printing method, and was baked in the air at 500°C for 3 hours. Then, a baked body of the titanium oxide, which was obtained, was immersed in 40 mM TiCl4 aqueous solution, and was heated at 60°C for 1 hour, and heating was continuously performed at 500°C for 30 minutes, thereby forming the porous layer 12 (film thickness: 250 nm) formed from $TiO_2$.

<Formation of Photosensitive Layer 13A>

A 40% methanol solution (10 g) of methyl amine, and an aqueous solution of 47% by mass of hydrogen bromide (hydrobromic acid: 15.6 g) were stirred in a flask at 0°C for 2 hours, and were concentrated to obtain coarse $CH_3NH_3Br$. The coarse $CH_3NH_3Br$ that was obtained was dissolved in ethanol and was recrystallized with diethylether. A precipitated crystal that was obtained was filtered and collected, and was dried under reduced pressure at 60°C for 5 hours, thereby obtaining purified $CH_3NH_3Br$.

Then, the purified $CH_3NH_3Br$ and $PbBr_2$ were stirred and mixed by a molar ratio of 3:1 in DMF at 60°C for 12 hours, and the resultant mixture was filtered with a polytetrafluoroethylene (PTFE) syringe filter, thereby preparing a 40% by mass light absorbing agent solution.

The prepared light absorbing agent solution A was applied onto the porous layer 12 formed on the conductive support 11 with a spin coating method (for 60 seconds at 2000 rpm), and the applied light absorbing agent solution A was dried by using a hot plate at 100°C for 60 minutes, thereby forming the photosensitive layer 13A (film thickness: 300 nm (including the film thickness of 250 nm of the porous layer 12)) including a perovskite compound of $CH_3NH_3PbBr_3$.

In this manner, the first electrode 1A was prepared.

Furthermore, in Sample No. 111, the photosensitive layer was formed by using a perovskite compound of $CH_3NH_3PbI_3$ instead of the perovskite compound of $CH_3NH_3PbBr_3$. The perovskite compound $CH_3NH_3PbI_3$ was obtained in the same manner as described above except that the material $CH_3NH_3Br$ was changed to $CH_3NH_3I$, and $PbBr_2$ was changed to $PbI_2$.

<Preparation of Hole Transporting Material Solution>

The compound a1 (180 mg) as the hole transporting material was dissolved in chlorobenzene (1 mL), thereby preparing a hole transporting material solution.

<Formation of Hole Transport Layer 3A>

Then, the prepared solution for the hole transport layer was applied onto a surface of the first electrode 1A with a spin coating method and was dried, thereby forming the solid-shaped hole transport layer 3A (film thickness: 100 nm).

<Preparation of Second Electrode 2>

Gold was vapor-deposited on the hole transport layer 3A with a vapor deposition method to prepare the second electrode 2 (film thickness: 100 nm).

In this manner, the photoelectric conversion element 10A (Sample No. 101) was manufactured.

Respective film thicknesses were measured through observation with a SEM according to the above-described method.

[Manufacturing of Photoelectric Conversion Elements (Sample Nos. 102 to 111, and c101 to c104)]

• Sample Nos. 102 to 110, and c101 to c103

Photoelectric conversion elements (Sample Nos. 102 to 110) of the present invention, and photoelectric conversion elements (Sample Nos. c101 to c103) for comparison were respectively manufactured in the same manner as in the manufacturing of the photoelectric conversion element (Sample No. 101) except that hole transporting material solutions respectively containing hole transporting materials illustrated in Table 1 were used instead of the hole transporting material a1 in comparison to the manufacturing of the photoelectric conversion element (Sample No. 101).

. Sample No. 111

A photoelectric conversion element (Sample No. 111) of the present invention was manufactured in the same manner as in the manufacturing of the photoelectric conversion element (Sample No. 101) except that a photosensitive layer containing the perovskite compound of $CH_3NH_3PbI_3$ was formed instead of the perovskite compound of $CH_3NH_3PbBr_3$ comparison to the manufacturing of the photoelectric conversion element (Sample No. 101).

• Sample No. c104

A photoelectric conversion element (Sample No. c104) for comparison was manufactured in the same manner as in the manufacturing of the photoelectric conversion element (Sample No. 101) except that hole transporting material illustrated in the following Table 1 was used instead of the hole transporting material a1, and preparation of the hole transporting material solution was changed as described below in comparison to the manufacturing of the photoelectric conversion element (Sample No. 101).

<Preparation of Hole Transporting Material Solution>

A hole transporting material (180 mg) illustrated in the following Table 1 was dissolved in chlorobenzene (1 mL). 37.5 μL of an acetonitrile solution obtained by dissolving lithium-bis (trifluoromethanesulfonyl) imide (170 mg) in acetonitrile (1 mL) and t-butyl pyridine (TBP, 17.5 μL) were additionally mixed to the chlorobenzene solution, thereby preparing a hole transporting material solution.

«Evaluation»

<Measurement of Initial Photoelectric Conversion Efficiency>

Initial photoelectric conversion efficiency was evaluated as follows.

Five specimens were manufactured for each of the photoelectric conversion elements of respective sample numbers. A battery characteristic test was performed with respect to each of the five specimens to measure initial photoelectric conversion efficiency ($\eta^{I}$/%) (after manufacturing). In addition, an average value of the five specimens was set as initial photoelectric conversion efficiency ($\eta^{IA}$/%) of the photoelectric conversion element of each sample number.

The battery characteristic test was performed through irradiation of pseudo-solar light of 1000 W/$m^2$ from a xenon lamp through an AMI.5 filter by using a solar simulator "WXS-85H" (manufactured by Wacom). Current-voltage characteristics were measured by using an I-V tester to obtain photoelectric conversion efficiency (η[%]).

It was confirmed that the specimens function as a solar cell when considering that a current flows in any of the specimens.

<Measurement of Time-Dependent Photoelectric Conversion Efficiency>

A time-dependent photoelectric conversion efficiency of the photoelectric conversion element was evaluated as follows.

Five specimens were manufactured for each of the photoelectric conversion elements of respective sample numbers. Each of the five specimens of photoelectric conversion elements was maintained in a constant-temperature and constant-humidity bath under a high-humidity environment of a temperature of 45°C and a relative humidity of 50%RH for 25 hours, and then a battery characteristic test was performed in the same manner as in <Measurement of Initial Photoelectric Conversion Efficiency> to measure photoelectric conversion efficiency ($\eta^{R}$/%) after the passage of

time. In addition, an average value of the five specimens was set as a time-dependent photoelectric conversion efficiency ($\eta^{RA}$/%) of the photoelectric conversion elements of the respective sample numbers.

<Evaluation of Moisture Resistance Variation>

A moisture resistance variation of the photoelectric conversion element was evaluated as follows.

First, with respect to the entirety of the five specimens of photoelectric conversion elements in each of the respective sample numbers, a deterioration rate of the photoelectric conversion efficiency was calculated in accordance with the following Expression (I).

$$\text{Deterioration rate (\%)} = 100 - \{100 \times [(\text{time-dependent photoelectric conversion efficiency } \eta^{R})/(\text{initial photoelectric conversion efficiency } \eta^{I})]\} \quad \text{(I)}$$

Next, an average deterioration rate of the photoelectric conversion efficiency of the five specimens of photoelectric conversion elements in each of the sample numbers was calculated in accordance with the following Expression (II).

$$\text{Average deterioration rate (\%)} = 100 - \{100 \times [(\text{time-dependent photoelectric conversion efficiency } \eta^{RA})/(\text{initial photoelectric conversion efficiency } \eta^{IA})]\} \quad \text{(II)}$$

The moisture resistance variation of the photoelectric conversion element was evaluated as follows. That is, when an average deterioration rate of the photoelectric conversion efficiency, which is calculated by Expression (II), is set as "1", ranges, in which the greatest difference of a relative deterioration rate from the average deterioration rate among five specimens is included, are classified under the following evaluation standard to evaluate the moisture resistance variation.

When subtracting 1 from a value obtained by dividing the deterioration rate of each of the five specimens by the average deterioration rate, "the greatest difference of the relative deterioration rate" represents a difference of which an absolute value is the greatest.

- Evaluation Standard of Moisture Resistance Variation -

A: Maximum value is in a range of $\pm$0.16
B: Maximum value is beyond a range of $\pm$0.16 and is in a range of $\pm$0.19
C: Maximum value is beyond a range of $\pm$0.19 and is in a range of $\pm$0.23
D: Maximum value is beyond a range of $\pm$0.23 and is in a range of $\pm$0.27
E: Maximum value is beyond a range of $\pm$0.27 and is in a range of $\pm$0.31
F: Maximum value is beyond a range of $\pm$0.31

Results are illustrated in the following Table 1. In the evaluation of the moisture resistance variation, [A], [B], [C], and [D] are passing levels, [A], [B], and [C] are preferable, and [A] is more preferable. On the other hand, in [E] and [F], moisture resistance variation between elements is irregular, and the moisture resistance does not reach the passing level (required level) of the present invention.

Furthermore, with respect to specimens which were separately prepared, Au was vapor-deposited to form a film in succession to the hole transport layer, and the battery performance was measured. From the measurement, it was confirmed that the specimens function as a solar cell when considering that a current flow in any of the specimens.

[Table 1]

| Sample No. | | Photosensitive layer | Hole transporting material | Moisture resistance variation |
|---|---|---|---|---|
| 101 | Present invention 1 | $CH_3NH_3PbBr_3$ | a1 | A |
| 102 | Present invention 2 | $CH_3NH_3PbBr_3$ | a2 | B |

(continued)

| Sample No. | | Photosensitive layer | Hole transporting material | Moisture resistance variation |
|---|---|---|---|---|
| 103 | Present invention 3 | $CH_3NH_3PbBr_3$ | a4 | B |
| 104 | Present invention 4 | $CH_3NH_3PbBr_3$ | a5 | C |
| 105 | Present invention 5 | $CH_3NH_3PbBr_3$ | a6 | C |
| 106 | Present invention 6 | $CH_3NH_3PbBr_3$ | a7 | A |
| 107 | Present invention 7 | $CH_3NH_3PbBr_3$ | a8 | D |
| 108 | Present invention 8 | $CH_3NH_3PbBr_3$ | a9 | A |
| 109 | Present invention 9 | $CH_3NH_3PbBr_3$ | a10 | A |
| 110 | Present invention 10 | $CH_3NH_3PbBr_3$ | a11 | D |
| 111 | Present invention 11 | $CH_3NH_3PbI_3$ | a1 | A |
| c101 | Comparative Example 1 | $CH_3NH_3PbBr_3$ | spiro-MeOTAD | F |
| c102 | Comparative Example 2 | $CH_3NH_3PbBr_3$ | b1 | E |
| c103 | Comparative Example 3 | $CH_3NH_3PbBr_3$ | b2 | E |
| c104 | Comparative Example 4 | $CH_3NH_3PbBr_3$ | spiro-MeOTAD | F |

As illustrated in Table 1, it could be seen that in the photoelectric conversion elements of the present invention which include a compound having the at least one structure represented by Formula 1 in the hole transport layer, it is possible to suppress the moisture resistance variation.

Explanation of References

1A to 1E: first electrode
11: conductive support
11a: support
11b: transparent electrode
12: porous layer
13A to 13C: photosensitive layer
14: blocking layer
2: second electrode
3A, 3B, 16: hole transport layer
4, 15: electron transport layer
6: external circuit (lead)
10A to 10E: photoelectric conversion element
100A to 100E: system using solar cell
M: electric motor

## Claims

**1.** A photoelectric conversion element comprising, at least:

a conductive support;
a photosensitive layer that contains a light absorbing agent;

a hole transport layer that contains a hole transporting material; and
a second electrode,
wherein at least one of the photosensitive layer or the hole transport layer is provided on the conductive support to constitute a first electrode in combination with the conductive support, and
wherein the hole transport layer contains a compound Q having at least one structure M represented by Formula 1 as the hole transporting material,
provided that in a case where the compound Q has two or more of the structures M, the two or more structures M may be the same as or different from each other,

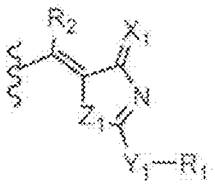

Formula 1

in Formula 1,

$Z_1$ represents a sulfur atom, a selenium atom, or an oxygen atom,
$Y_1$ represents NR, a sulfur atom, a selenium atom, or an oxygen atom,
$X_1$ represents NR, a sulfur atom, an oxygen atom, a selenium atom, or $CR_3R_4$,
$R_1$ represents a substituent,
$R_2$, $R_3$, $R_4$, and R each independently represent a hydrogen atom or a substituent, and
a wavy line represents a binding site with a remainder of the compound Q.

**2.** The photoelectric conversion element according to claim 1,
wherein the hole transport layer contains at least a compound Q represented by Formula 2 as the hole transporting material,

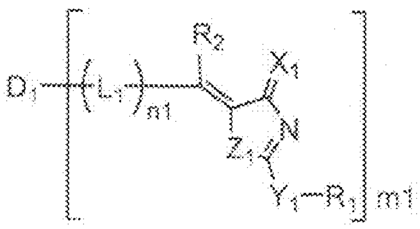

Formula 2

in Formula 2,

$Z_1$ represents a sulfur atom, a selenium atom, or an oxygen atom,
$Y_1$ represents NR, a sulfur atom, a selenium atom, or an oxygen atom,
$X_1$ represents NR, a sulfur atom, an oxygen atom, a selenium atom, or $CR_3R_4$,
$R_1$ represents a substituent,
$R_2$, $R_3$, $R_4$, and R each independently represent a hydrogen atom or a substituent,
$L_1$ represents a linking group,
$D_1$ represents an organic group or a nitrogen atom, the organic group represented by $D_1$ may have another substituent in Formula 2 that is different from a substituent represented by Formula 2a, and the nitrogen atom may have another substituent that is different from a substituent represented by Formula 2a or a hydrogen atom, $Z_1$, $Y_1$, $X_1$, $R_1$, $R_2$, $L_1$, n1, and m1 in Formula 2a are the same as $Z_1$, $Y_1$, $X_1$, $R_1$, $R_2$, $L_1$, n1, and m1 in Formula 2,
n1 represents an integer of 0 to 11, and in a case where n1 is an integer of 2 or more, a plurality of $L_1$'s may be the same as or different from each other,
in a case where $D_1$ is an organic group, m1 represents an integer of 1 to 4, in a case where $D_1$ is a nitrogen atom, m1 represents an integer of 1 to 3, and in a case where m1 is an integer of 2 or more, a plurality of $Z_1$'s, $Y_1$'s, $X_1$'s, $R_1$'s, and $R_2$'s may be the same as or different from each other, respectively.

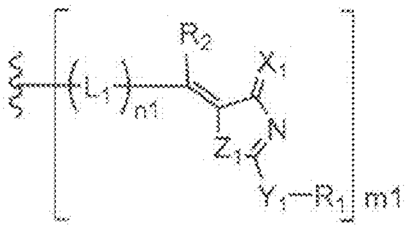

Formula 2a

3. The photoelectric conversion element according to claim 1 or 2, wherein the hole transport layer contains at least a compound Q represented by Formula 3 as the hole transporting material,

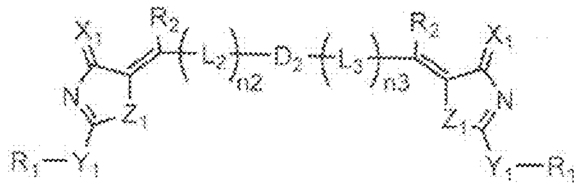

Formula 3

in Formula 3,

$Z_1$'s each independently represent a sulfur atom, a selenium atom, or an oxygen atom,
$Y_1$'s each independently represent NR, a sulfur atom, a selenium atom, or an oxygen atom,
$X_1$'s each independently represent NR, a sulfur atom, an oxygen atom, a selenium atom, or $CR_3R_4$,
$R_1$'s each independently represent a substituent,
$R_2$, $R_3$, $R_4$, and R each independently represent a hydrogen atom or a substituent,
$L_2$ and $L_3$ each independently represent an alkenylene group, a cycloalkenylene group, an alkynylene group, an arylene group, or a heteroarylene group,
$D_2$ represents a linking group,
n2 and n3 each independently represent an integer of 1 to 5, in a case where n2 is an integer of 2 to 5, a plurality of $L_2$'s may be the same as or different from each other, and in a case where n3 is an integer of 2 to 5, a plurality of $L_3$'s may be the same as or different from each other.

4. The photoelectric conversion element according to claim 3, wherein $D_2$ in Formula 3 represents a divalent fused ring group containing at least one aromatic ring or a divalent fused ring group containing at least one aromatic hetero ring.

5. The photoelectric conversion element according to any one of claims 1 to 4, wherein the at least one $X_1$ is an oxygen atom.

6. The photoelectric conversion element according to any one of claims 1 to 5, wherein the at least one $Z_1$ is a sulfur atom.

7. The photoelectric conversion element according to any one of claims 1 to 6, wherein the at least one $Y_1$ is a sulfur atom.

8. The photoelectric conversion element according to any one of claims 1 to 7, wherein the photosensitive layer contains at least a compound having a perovskite-type crystal structure that has a cation of an element of Group 1 in the periodic table or a cationic organic group A, a cation of a metal atom M other than elements of Group 1 in the periodic table, and an anion of an anionic atom or atomic group X as the light absorbing agent.

9. A solar cell, comprising: the photoelectric conversion element according to any one of claims 1 to 8.

10. A compound represented by Formula 3,

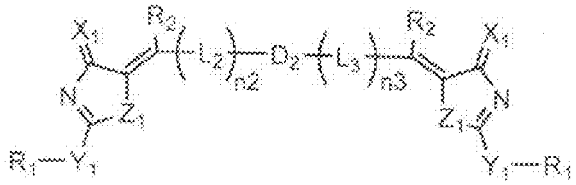

Formula 3

in Formula 3,

$Z_1$'s each independently represent a sulfur atom, a selenium atom, or an oxygen atom,
$Y_1$'s each independently represent NR, a sulfur atom, a selenium atom, or an oxygen atom,
$X_1$'s each independently represent NR, a sulfur atom, an oxygen atom, a selenium atom, or $CR_3R_4$,
$R_1$'s each independently represent a substituent,
$R_2$, $R_3$, $R_4$, and R each independently represent a hydrogen atom or a substituent,
$L_2$ and $L_3$ each independently represent an alkenylene group, a cycloalkenylene group, an alkynylene group, an arylene group, or a heteroarylene group,
$D_2$ represents a linking group,
n2 and n3 each independently represent an integer of 1 to 5, in a case where n2 is an integer of 2 to 5, a plurality of $L_2$'s may be the same as or different from each other, and in a case where n3 is an integer of 2 to 5, a plurality of $L_3$'s may be the same as or different from each other.

**11.** The compound according to claim 10,
wherein $D_2$ in Formula 3 represents a divalent fused ring group containing at least one aromatic ring or a divalent fused ring group containing at least one aromatic hetero ring.

FIG. 1A

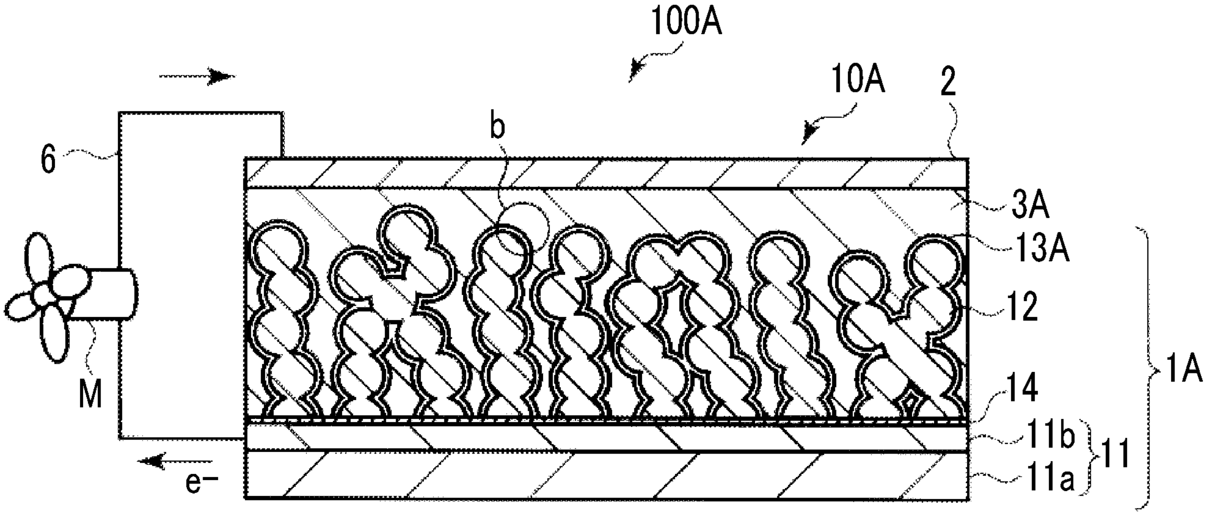


FIG. 1B

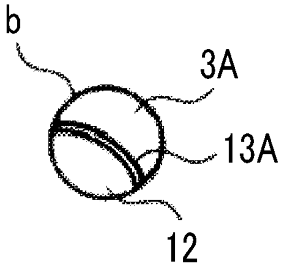


FIG. 2

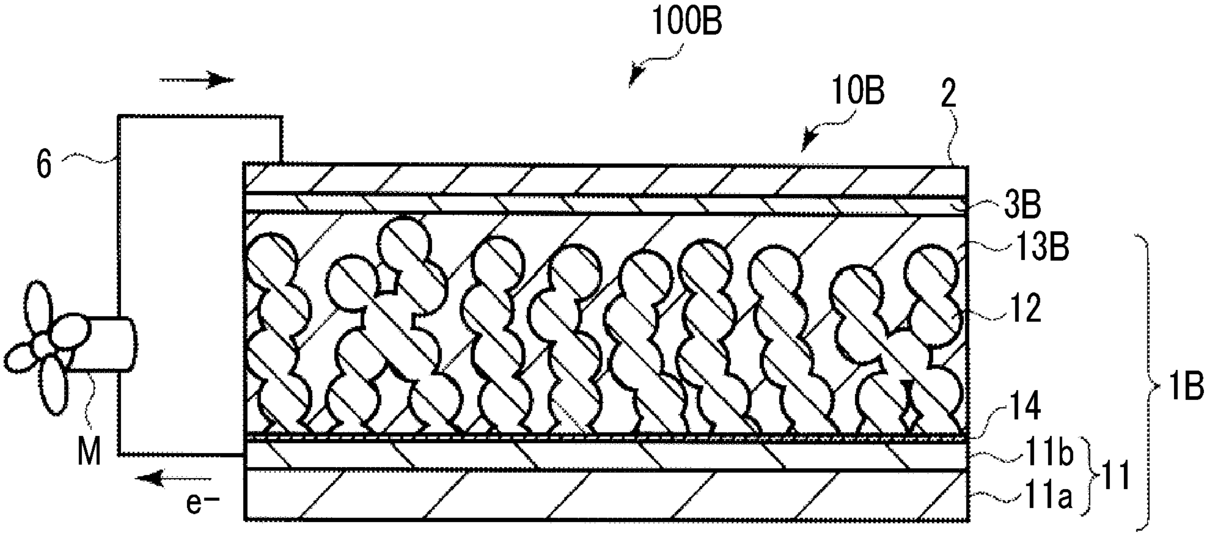

FIG. 3

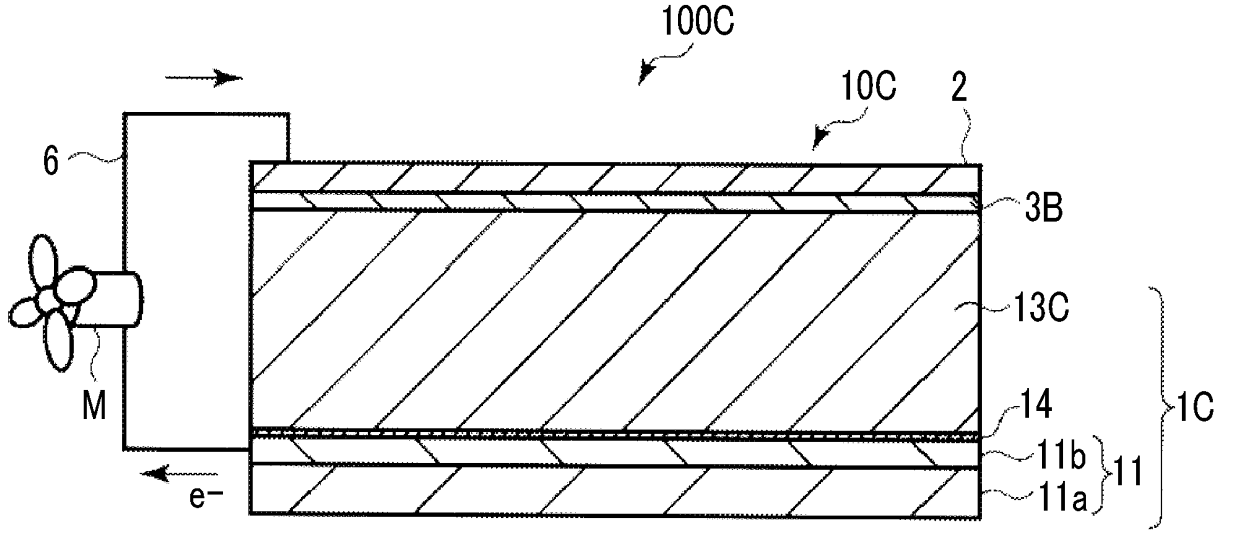
100C
10C
2
6
3B
13C
M
14
1C
11b
11
e−
11a

FIG. 4

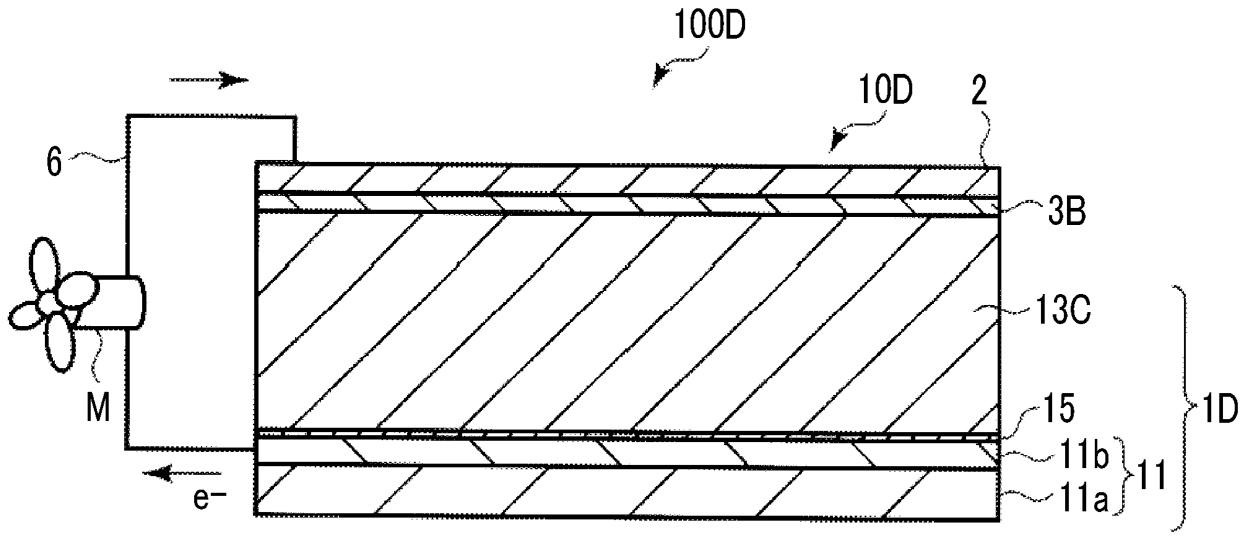
100D
10D
2
6
3B
13C
M
15
1D
11b
11
e−
11a

FIG. 5

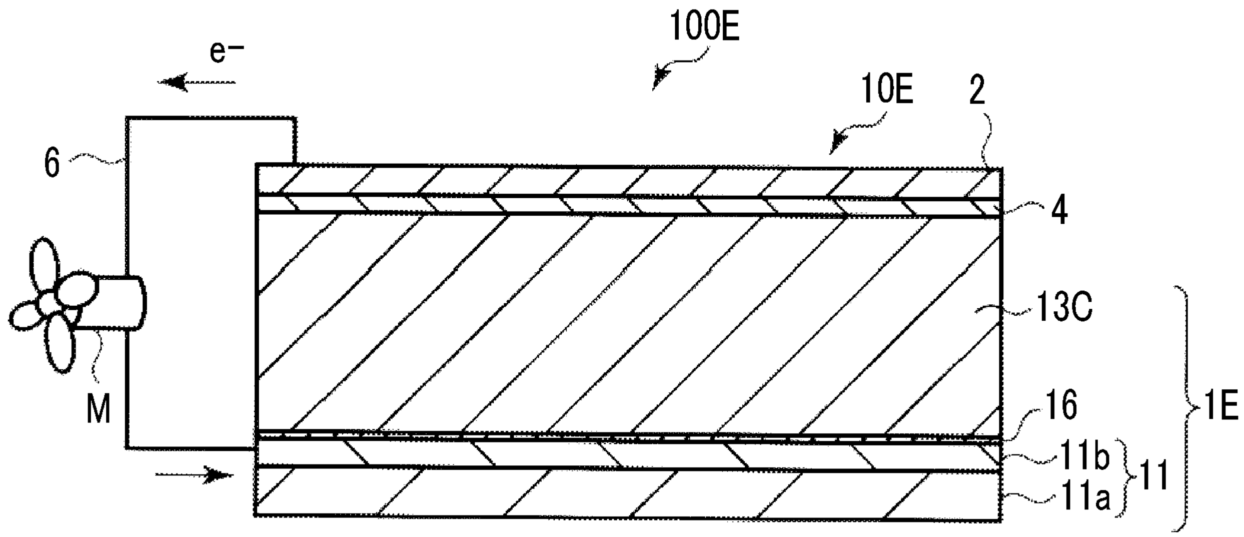

## INTERNATIONAL SEARCH REPORT

International application No. PCT/JP2017/005393

A. CLASSIFICATION OF SUBJECT MATTER
*H01L51/46*(2006.01)i, *H01L51/44*(2006.01)i, *C07D495/04*(2006.01)n

According to International Patent Classification (IPC) or to both national classification and IPC

B. FIELDS SEARCHED

Minimum documentation searched (classification system followed by classification symbols)
H01L51/46, H01L51/44, C07D495/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2007-47770 A (Fujifilm Corp.), 22 February 2007 (22.02.2007), paragraphs [0054], [0252] to [0330] (Family: none) | 1-11 |
| A | L. ZHENG et al., A hydrophobic hole transporting oligothiophene for planar perovskite solar cells with improved stability, CHEMICAL COMMUNICATIONS, Vol.50, No.76, 2014.10.04, pp.11196-11199 | 1-11 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 April 2017 (27.04.17) | 16 May 2017 (16.05.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2017/005393

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

(Invention 1) claims 1-9

(Continued to extra sheet)

1. ☒ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (January 2015)

INTERNATIONAL SEARCH REPORT

International application No.
PCT/JP2017/005393

Continuation of Box No.III of continuation of first sheet(2)

Since the invention of claims 1-9 has a special technical feature that is "a photoelectric conversion element which is provided at least with a conductive supporting body, a photosensitive layer containing a light absorbent, a hole transport layer containing a hole transport material, and a second electrode, and wherein: at least one of the photosensitive layer and the hole transport layer is provided on the conductive supporting body so as to constitute a first electrode together with the conductive supporting body; and the hole transport layer contains, as the hole transport material, a compound Q that has at least one structure M represented by formula 1", the invention of claims 1-9 is placed in Invention 1.

(Invention 2) claims 10 and 11

Claims 10 and 11 and claim 1 placed in Invention 1 have a common technical feature that is "a structure M represented by formula 1".

However, the above-said technical feature cannot be considered to be a special technical feature, since the technical feature does not make a contribution over the prior art in the light of the contents disclosed in the document 1 (see JP 2007-47770 A, paragraph [0054], chemical formula 8; paragraph [0055], chemical formula 9).

Further, there is no other same or corresponding special technical feature between these inventions.

In addition, claims 10 and 11 are not dependent on claim 1.

Further, claims 10 and 11 have no relationship such that these claims are substantially same as or equivalent to any claim classified into Invention 1.

Consequently, claims 10 and 11 cannot be classified into Invention 1.

Since claims 10 and 11 have a special technical feature that is "a compound represented by formula 3", claims 10 and 11 are placed in Invention 2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013109965 A
- JP 2001291534 A
- US 4927721 A
- US 4684537 A
- US 5084365 A
- US 5350644 A
- US 5463057 A
- US 5525440 A
- JP 7249790 A
- JP H7249790 A
- JP 2004220974 A
- JP 2008135197 A
- JP 2005135902 A
- JP 2001160425 A
- JP 2003123859 A
- JP 2002260746 A


### Non-patent literature cited in the description


- *Science,* 2012, vol. 388, 643-647
- *J. Mater. Chem. A,* 2015, vol. 3, 11940-11947
- *Energy Environ. Sci.,* 2014, vol. 7, 2981-2985
- *J. Am. Chem. Soc.,* 2009, vol. 131 (17), 6050-6051
- *Science,* 2012, vol. 338, 643-647
- **AKIHIRO KOJIMA ; KENJIRO TESHIMA ; YASUO SHIRAI ; TSUTOMU MIYASAKA.** Organometal Halide Perovskites as Visible-Light Sensitizers for Photovoltaic Cells. *J. Am. Chem. Soc.,* 2009, vol. 131 (17), 6050-6051
- *J. Org. Chem.,* 2014, vol. 79, 7766
- *Journal of Medicinal Chemistry,* 2007, vol. 50, 1546
- *European Journal of Organic Chemistry,* 2007, vol. 425
- *Org. Lett.,* 2005, vol. 7, 5253
- *Macromolecules,* 2003, vol. 36, 7986
- *Chemistry of Materials,* 2010, vol. 22, 1977
- *Angewandte Chem-International Edition,* 2006, vol. 45, 3170
- *Tetorahedron,* 2012, vol. 68, 767
- *Org. Lett.,* 2012, vol. 14, 4330
- *Tetorahedron Letters,* 2012, vol. 53, 5197
- *Journal of Materials Chemistry C,* 2014, vol. 2, 124
- *J. Org. Chem.,* 2010, vol. 75, 495
- *European Journal of Organic Chemistry,* 2013, 1916
- *Journal of American Chemical Society,* 2012, vol. 134, 11064
- *Journal of Materials Chemistry A,* 2013, vol. 1, 2795
- *Journal of Materials Chemistry,* 2012, vol. 22, 9173
- *J. Am. Chem. Soc.,* 2009, vol. 131 (17), 6050-6051
- *Science,* 2012, vol. 338, 643
- *Chemical Review,* 2010, vol. 110, 6595